(19) Europäisches Patentamt / European Patent Office / Office européen des brevets

(11) **EP 4 497 393 A1**

(12) **EUROPEAN PATENT APPLICATION**
published in accordance with Art. 153(4) EPC

(43) Date of publication:
**29.01.2025 Bulletin 2025/05**

(21) Application number: **23779290.8**

(22) Date of filing: **07.03.2023**

(51) International Patent Classification (IPC):
***A61B 8/12*** (2006.01)

(52) Cooperative Patent Classification (CPC):
**A61B 8/12**

(86) International application number:
**PCT/JP2023/008441**

(87) International publication number:
**WO 2023/189262 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.03.2022 JP 2022061024**

(71) Applicant: **Terumo Kabushiki Kaisha Tokyo 151-0072 (JP)**

(72) Inventors:
• **SHIMIZU, Hijiri**
  **Ashigarakami-gun, Kanagawa 259-0151 (JP)**
• **INOUE, Koichi**
  **Ashigarakami-gun, Kanagawa 259-0151 (JP)**
• **MATSUSHITA, Yujiro**
  **Ashigarakami-gun, Kanagawa 259-0151 (JP)**

(74) Representative: **Casalonga**
  **Casalonga & Partners**
  **Bayerstraße 71/73**
  **80335 München (DE)**

(54) **PROGRAM, INFORMATION PROCESSING METHOD, AND INFORMATION PROCESSING DEVICE**

(57)    To provide a program and the like capable of generating an image including information on the size of an imaging target.

The program causes a computer to execute processing involving: acquiring a plurality of tomographic images (41) generated by three-dimensional scanning using an image-acquiring catheter that acquires an image while moving a scanning plane in an axial direction; determining a representative line (42) for each of the tomographic images (41); calculating an index value of a scanning line in a calculation range determined based on an intersection between the scanning line forming each of the tomographic images (41) and the representative line (42); and displaying a color determined based on the index value at a coordinate determined by a position of each of the tomographic images (41) and a position of the intersection.

*FIG. 1*

EP 4 497 393 A1

**Description**

Technical Field

The present invention relates to a program, an information processing method, and an information processing apparatus.

Background Art

Patent Literature 1 proposes a diagnosis support device that generates a two-dimensional image based on a batch of tomographic images acquired with a three-dimensional scanning intravascular ultrasound (IVUS) catheter. In the generated image, similarities between a brightness pattern of each scanning line and a predefined pattern classification are arranged by two axes, the angle of each scanning line and the axial direction of the catheter.

Citation List

Patent Literature

Patent Literature 1: JP 2021-41029 A

Summary of Invention

Technical Problem

In an image generated by the diagnosis support device disclosed in Patent Literature 1, a blood vessel can be seen as if it is incised, which enables a user to check, for example, how strands of a stent implanted inside the blood vessel are placed.
However, the image generated by the device disclosed in Patent Literature 1 has a difficulty in evaluating the absolute size of an imaging target included in a scanning line. For example, a plaque area, a degree of expansion of the stent, the change in diameter of the blood vessel, and the like cannot be observed in the image generated by the device disclosed in Patent Literature 1.
In an aspect, an object is to provide a program or the like capable of generating an image including information on the size of an imaging target.

Solution to Problem

A program causes a computer to execute processing involving: acquiring a plurality of tomographic images generated by three-dimensional scanning using an image-acquiring catheter that acquires an image while moving a scanning plane in an axial direction; determining a representative line for each of the tomographic images; calculating an index value of a scanning line in a calculation range determined based on an intersection between the scanning line forming each of the tomographic images and the representative line; and displaying a color determined based on the index value at a coordinate determined by a position of each of the tomographic images and a position of the intersection.

Advantageous Effects of Invention

In an aspect, there is provided a program or the like capable of generating an image including information on the size of an imaging target.

Brief Description of Drawings

Fig. 1 is a view for describing an outline of an image generation method.
Fig. 2 is a view for describing a configuration of an information processing apparatus.
Fig. 3 is a view for describing a method of generating an index value stripe.
Fig. 4A is a view for describing a method of calculating an index value example-1.
Fig. 4B is a view for describing the method of calculating the index value example-1.
Fig. 5A is a view for describing the method of calculating the index value example-1.

Fig. 5B is a view for describing a modification of the method of calculating the index value example-1.

Fig. 6A is a view for describing a method of calculating an index value example-2.

Fig. 6B is a view for describing the method of calculating the index value example-2.

Fig. 7A is a view for describing a method of calculating an index value example-3.

Fig. 7B is a view for describing the method of calculating the index value example-3.

Fig. 8 is a view for describing the method of calculating the index value example-3.

Fig. 9A is a view for describing a method of converting between an angle and a length.

Fig. 9B is a view for describing the method of converting between an angle and a length.

Fig. 9C is a view for describing the method of converting between an angle and a length.

Fig. 10 is a view for describing the method of converting between an angle and a length.

Fig. 11 is a view for describing a method of determining a representative line.

Fig. 12 is a view for describing the method of determining a representative line.

Fig. 13A is a view for describing a record layout of a tomographic image DB.

Fig. 13B is a view for describing a record layout of a maximum gradient point DB.

Fig. 13C is a view for describing a record layout of a representative line DB.

Fig. 14 is a view for describing a record layout of an index value DB.

Fig. 15 is a flowchart for describing a processing flow of a program.

Fig. 16 is a flowchart for describing a processing flow of a subroutine for temporary representative line calculation.

Fig. 17 is a flowchart for describing a processing flow of a subroutine for representative line calculation.

Fig. 18 is a flowchart for describing a processing flow of a subroutine for index value calculation.

Fig. 19 is a flowchart for describing a processing flow of a subroutine for first index value calculation.

Fig. 20 is a flowchart for describing a processing flow of a subroutine for second index value calculation.

Fig. 21 is a flowchart for describing a processing flow of a subroutine for third index value calculation.

Fig. 22 is a flowchart for describing a processing flow of a subroutine for display.

Fig. 23A is a view for describing a relation between an angle and an area according to a modification.

Fig. 23B is a view for describing the relation between an angle and an area according to the modification.

Fig. 23C is a view for describing the relation between an angle and an area according to the modification.

Fig. 24 is an example of a screen according to a second embodiment.

Fig. 25 is a view for describing a configuration of a catheter system according to a third embodiment.

Fig. 26 is a view for describing a configuration of an information processing apparatus according to a fourth embodiment.

Description of Embodiments

[First Embodiment]

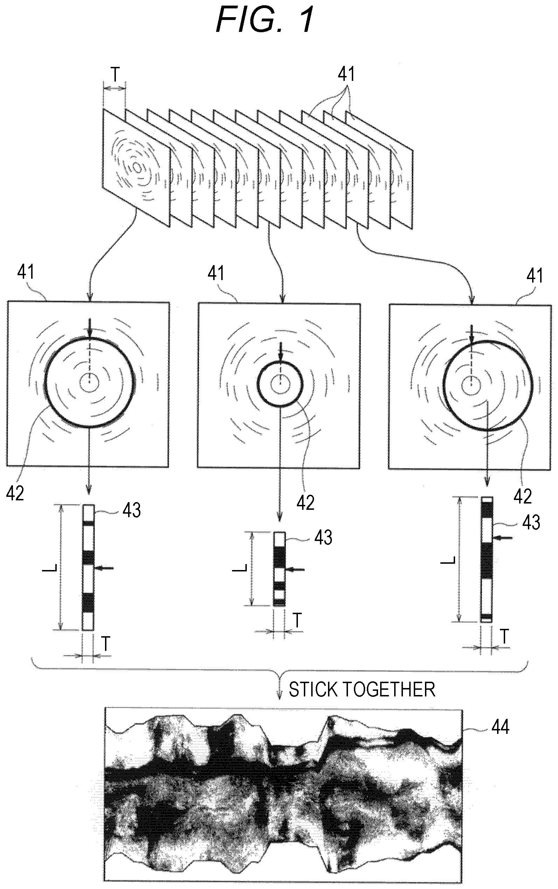

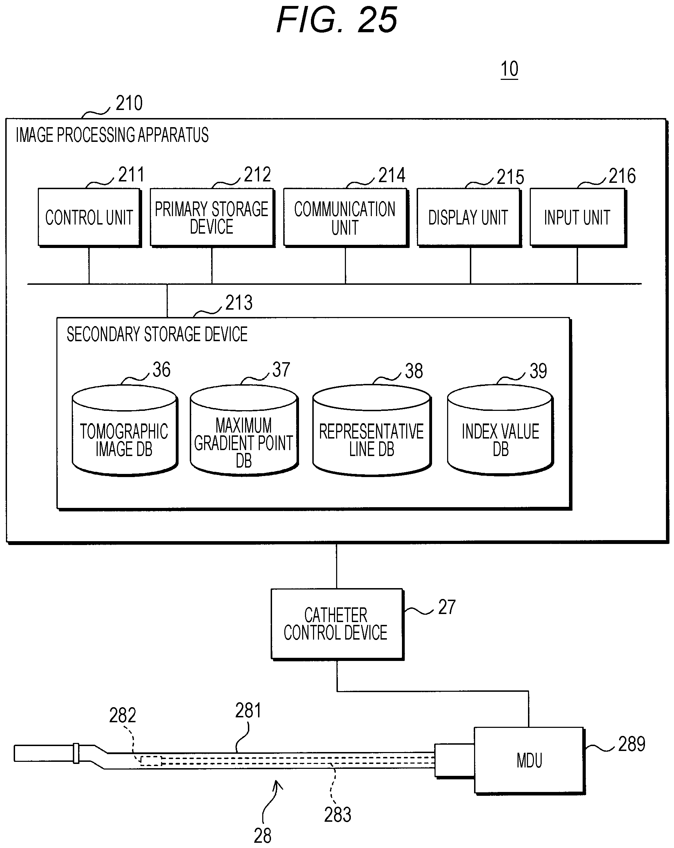

Fig. 1 is a view for describing an outline of an image generation method. A user such as a doctor inserts an image-acquiring catheter 28 (see Fig. 25) into a part near an affected part. The image-acquiring catheter 28 is used for three-dimensional scanning and capable of continuously capturing a plurality of tomographic images 41 by gradually changing scanning planes in an axial direction.

An interval between the tomographic images 41 is denoted by an interval T. Hereinafter, the tomographic images 41 created by one three-dimensional scanning may be referred to as a batch of tomographic images 41.

A representative line 42 is determined for each tomographic image 41. The representative line 42 is a closed curve in a tomographic image 41 determined based on, for example, the depth where a stent is implanted, the external elastic lamina of a blood vessel into which the image-acquiring catheter 28 is inserted, or the depth where the brightness greatly changes. The representative line 42 may be a closed curve designated by the user.

Hereinafter illustrated is a case where the representative line 42 has a circular shape surrounding the image-acquiring catheter 28. The center position and the radius of the representative line 42 are determined for each tomographic image 41. A specific example of how to determine the representative line 42 will be described later.

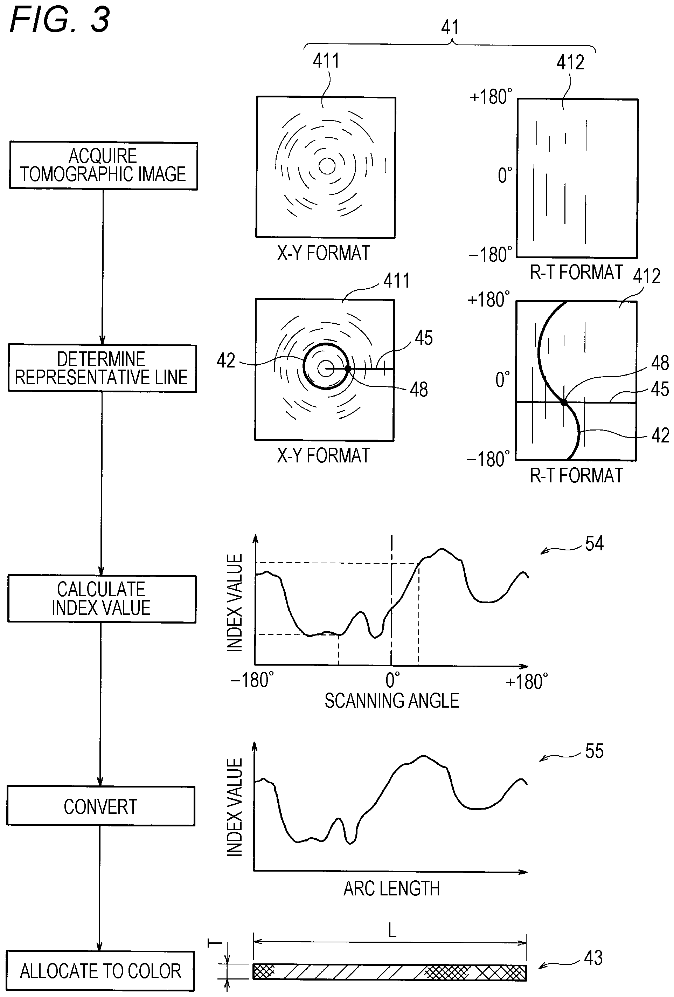

For each scanning line 45 (see Fig. 3) that forms a tomographic image 41, an index value is calculated. The index value is a constant calculated based on characteristics of a scanning line 45 and a position of an intersection 48 (see Fig. 3) on the scanning line 45. A specific example of how to calculate the index value will be described later.

The index value calculated for each scanning line 45 is allocated to the intersection 48 between each scanning line 45 and the representative line 42, thereby creating an index value stripe 43. The index value stripe 43 is a virtual band formed by cutting the representative line 42, which is colored based on the index value, at one spot on the lower side of Fig. 1, that is, a position scanned at a scanning angle of 180 degrees, and by stretching the representative line 42. Note that a region where the intersection 48 has a low density is interpolated by a known interpolation technique such as linear interpolation.

The index value stripe 43 has the same width as the interval T between the tomographic images 41 and the same length as a length L of the representative line 42. In Fig. 1, short arrows in contact with each representative line 42 and each index value stripe 43 indicate a position directly above the image-acquiring catheter 28 in each tomographic image 41, that is, a position scanned at a scanning angle of 0 degrees. Details on how to generate the index value stripe 43 will be described later.

Index value stripes 43 corresponding to respective tomographic images 41 are arranged in such a manner that, for example, positions scanned at a scanning angle of 0 degrees are aligned in a straight line, thereby generating an index value image 44. The index value image 44 is a flat surface image obtained by mapping index values on the surface of a solid formed by representative lines 42 corresponding to respective tomographic images 41 and by cutting the representative lines 42 open at positions scanned at a scanning angle of 180 degrees.

The index value image 44 enables the user to intuitively understand the size of the representative lines 42 and index values around depths of the representative lines 42 and to diagnose and provide medical treatment.

A position where each representative line 42 is cut open to form one index value stripe 43 is not limited to the position scanned at a scanning angle of 180 degrees. For example, in a case where a region of interest, or an affected part, is visualized on the lower side of a tomographic image 41, if each representative line 42 is cut open at a position scanned at an angle of 180 degrees, the affected part is separated and shown in upper and lower parts of the index value image 44. In contrast, if index value stripes 43 having representative lines 42 cut open at positions scanned at a scanning angle of 0 degrees are arranged in such a manner that positions scanned at a scanning angle of 180 degrees are aligned in a straight line, it is possible to generate the index value image 44 where the region of interest is easily observed.

Similarly, a reference scanning angle for arranging the index value stripes 43 is not limited to the position scanned at a scanning angle of 0 degrees or 180 degrees. In a case where representative lines 42 are arranged in such a manner that scanning angles corresponding to regions including the region of interest are aligned in a straight line, it is possible to generate the index value image 44 where a region scanned at an angle of interest is easily observed.

The user may appropriately select a scanning angle at which representative lines 42 are cut open and a scanning angle serving as the reference when arranging the index value stripes 43.

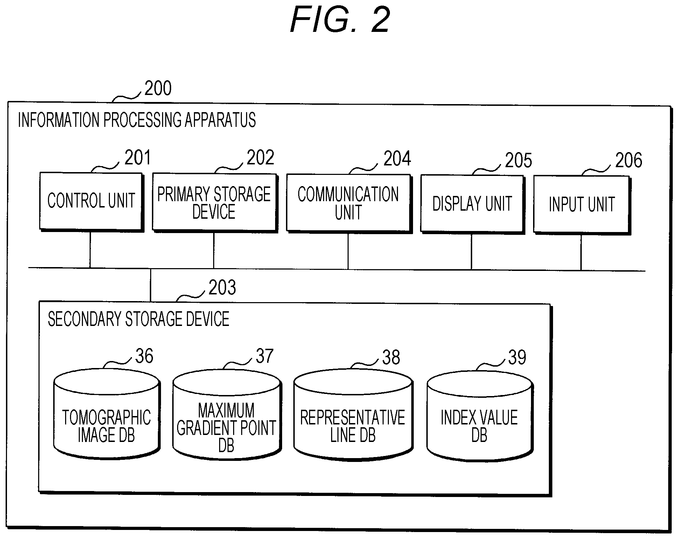

Fig. 2 is a view for describing a configuration of an information processing apparatus 200. The information processing apparatus 200 includes a control unit 201, a primary storage device 202, a secondary storage device 203, a communication unit 204, a display unit 205, an input unit 206, and a bus. The control unit 201 is an arithmetic control device that executes a program according to this embodiment. The control unit 201 employs one or more central processing units (CPUs) or graphics processing units (GPUs), or a multi-core CPU. Through the bus, the control unit 201 is connected to each hardware component constituting the information processing apparatus 200.

The primary storage device 202 is a storage device such as a static random access memory (SRAM), a dynamic random access memory (DRAM), and a flash memory. The primary storage device 202 temporarily stores information necessary during processing by the control unit 201 and a program being executed by the control unit 201.

The secondary storage device 203 is a storage device such as an SRAM, a flash memory, a hard disk, and a magnetic tape. The secondary storage device 203 stores a tomographic image database (DB) 36, a maximum gradient point DB 37, a representative line DB 38, an index value DB 39, programs to be executed by the control unit 201, and various kinds of data for executing the programs. The tomographic image DB 36, the maximum gradient point DB 37, the representative line DB 38, and the index value DB 39 may be stored in an external mass-storage device connected to the information processing apparatus 200. The communication unit 204 is an interface that conducts communication between the information processing apparatus 200 and a network.

The display unit 205 is, for example, a liquid crystal display panel or an organic electro-luminescence (EL) panel. The input unit 206 is, for example, a keyboard or a mouse. The display unit 205 and the input unit 206 may be layered to constitute a touch panel.

The information processing apparatus 200 is a general-purpose personal computer, a tablet, a large computing machine, a virtual machine that runs on a large computing machine, or a quantum computer. The information processing apparatus 200 may include a plurality of decentralized personal computers or hardware such as a large computing machine. The information processing apparatus 200 may include a cloud computing system. The information processing apparatus 200 may include a plurality of personal computers that operates simultaneously or hardware such as a large computing machine.

Fig. 3 is a view for describing a method of generating the index value stripe 43. With reference to Fig. 3, hereinafter described is how to generate one index value stripe 43 based on one tomographic image 41.

The tomographic image 41 can be expressed by both an X-Y format tomographic image 411 and an R-T format tomographic image 412. The X-Y format tomographic image 411 is an image constructed according to the actual shape. The center of the X-Y format tomographic image 411 corresponds to the center of the image-acquiring catheter 28. The R-T format tomographic image 412 is an image constructed by arranging scanning lines 45 in parallel in the order of scanning angles. The left end of the R-T format tomographic image 412 corresponds to the center of the image-acquiring catheter 28.

Each scanning line 45 is represented by brightness data in which a distance from the center of the image-acquiring catheter 28 is associated with brightness of the tomographic image 41 in this distance. A method of calculating brightness data for each scanning line 45 using the image-acquiring catheter 28, a method of creating the tomographic image 41 using the brightness data, and a method of converting between R-T format and X-Y format are all known, and the details thereof will be omitted.

Hereinafter illustrated is a case where one tomographic image 41 is formed by scanning lines 45 having scanning angles from -180 degrees to 180 degrees. The description will be continued, defining that the scanning angle of 0 degrees is measured in upward direction, scanning angles measured clockwise are positive, and scanning angles measured counterclockwise are negative in the X-Y format tomographic image 411.

The control unit 201 acquires one tomographic image 41. The control unit 201 determines the representative line 42 based on the tomographic image 41. In the X-Y format tomographic image 411, the representative line 42 is a circle surrounding the image-acquiring catheter 28, and in the R-T format tomographic image 412, the representative line 42 is a curve extending from the upper end to the lower end of the image.

With regard to a scanning line 45, in the X-Y format tomographic image 411, it is a straight line extending radially from the center of the image, and in the R-T format tomographic image 412, it is a horizontal line. Fig. 3 illustrates a scanning line 45 having a scanning angle of 90 degrees. In both of the X-Y format tomographic image 411 and the R-T format tomographic image 412, one scanning line 45 intersects with the representative line 42 in one spot. Therefore, there is one intersection 48 for one scanning line 45.

The control unit 201 calculates an index value for each scanning line 45 based on a procedure to be described later. An index value example is illustrated in an angle-index value graph 54. The scanning angle $\theta$ of each scanning line 45 is taken along the abscissa in the angle-index value graph 54, and the minimum is -180 degrees while the maximum is 180 degrees. The index value is taken along the ordinate of the scanning line 45. As will be described later, index values are defined in various ways. The unit of index values varies depending on definition.

The control unit 201 converts the abscissa of the angle-index value graph 54 into an arc length of the representative line 42. The converted state is schematically illustrated in a position-index value graph 55. The arc length of the representative line 42 from a position scanned at a scanning angle of 0 degrees to a position scanned at a scanning angle of $\theta$ is taken along the abscissa in the position-index value graph 55. The index value is taken along the ordinate in the position-index value graph 55. Details of the conversion method will be described later.

The control unit 201 allocates, to a color, a magnitude of the index value at each position on the abscissa in the position-index value graph 55, thereby generating the index value stripe 43. The color may be either achromatic between white and black or chromatic. A specific example of how to allocate a magnitude to a color will be described later.

Through these steps, the control unit 201 generates one index value stripe 43 based on each tomographic image 41 constituting one batch of tomographic images 41. The control unit 201 generates the index value image 44 based on a plurality of index value stripes 43 as described with reference to Fig. 1.

[Index Value Example-1]

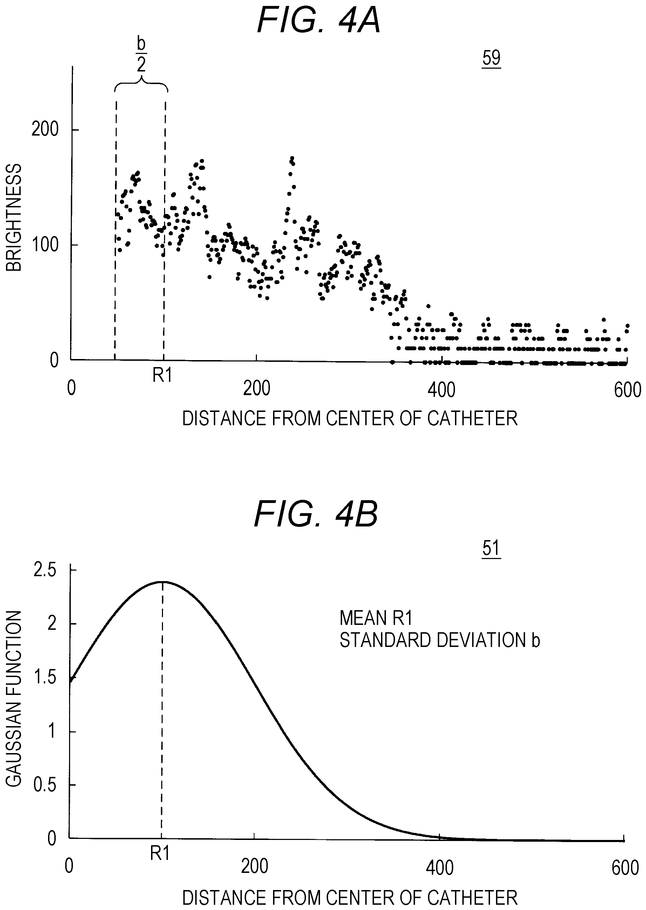

An index value example calculated with a Gaussian function will be described. Figs. 4A and 4B are views for describing a method of calculating an index value example-1. Fig. 4A illustrates a brightness graph 59 representing brightness data included in one scanning line 45. In the brightness graph 59, the distance from the center of the image-acquiring catheter 28 is taken along the abscissa.

In the following description, the distance from the center of the image-acquiring catheter 28 is indicated by the quantity of data on the brightness graph 59. That is to say, the unit of the abscissa in the brightness graph 59 is the quantity of data.

The brightness is taken along the ordinate in the brightness graph 59. The unit of the ordinate is a brightness value normalized to an integer from 0 to 255. In the following description, the x-th brightness data from the center of the image-acquiring catheter 28 is denoted by B(x).

R1 on the abscissa denotes a position where the scanning line 45 shown in the brightness graph 59 intersects the representative line 42. The symbol b is a constant. The constant b is defined by, for example, Formula (1).

[Math. 1]

$$b = \mathrm{Mag} \frac{P\,\mathrm{eff}}{D\,\mathrm{ep}} \quad \cdots\cdots \quad (1)$$

Mag is the fixed scaling factor.
Peff is the number of effective pixels.
Dep is the depth where an image is acquired.

The control unit 201 may receive an instruction from the user to change Mag. Formula (1) is an example of definition of the constant b. The constant b may be a value appropriately designated by the user. In Fig. 4A, note that data points are not illustrated in a region less than (R1 - b/2) on the abscissa.

Fig. 4B illustrates a Gaussian function graph 51 in which R1 denotes the mean and b denotes the standard deviation. Similarly to the brightness graph 59 illustrated in Fig. 4A, the distance from the center of the image-acquiring catheter 28 is taken along the abscissa in the Gaussian function graph 51. The Gaussian function is taken along the ordinate in the Gaussian function graph 51. A value N(x) of the Gaussian function corresponding to the x-th data from the center of the image-acquiring catheter 28 is calculated by Formula (2).

[Math. 2]

$$N(x) = \frac{1}{\sqrt{2\pi b}} \exp\left\{-\frac{(x - R1)^2}{2b^2}\right\} \quad \cdots \cdots \quad (2)$$

The peak of the Gaussian function graph 51 corresponds to the position of the intersection 48 between the scanning line 45 and the representative line 42. The mean R1 and the standard deviation b are examples of a first statistic.

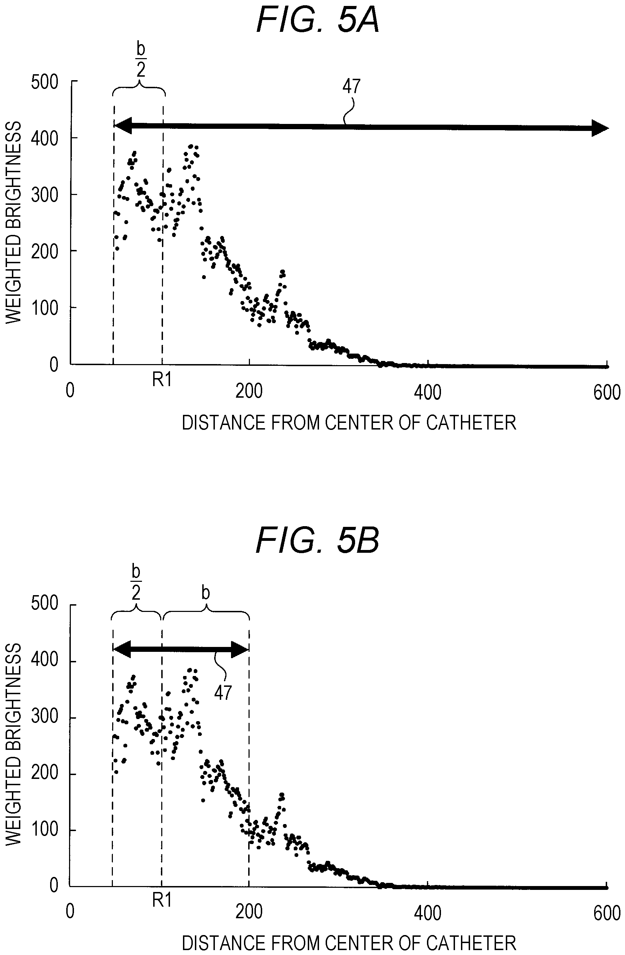

Fig. 5A is a view for describing the method of calculating the index value example-1. Fig. 5A is a graph illustrating a state where the brightness illustrated in Fig. 4A is weighted by the Gaussian function illustrated in Fig. 4B. Similarly to the brightness graph 59 illustrated in Fig. 4A, the distance from the center of the image-acquiring catheter 28 is taken along the abscissa in Fig. 5A. The weighted brightness is taken along the ordinate in Fig. 5A. Weighted brightness Bw(x) corresponding to the x-th data from the center of the image-acquiring catheter 28 is calculated by Formula (3).

$$Bw(x) = N(x) \cdot B(x) \quad \cdots \cdots \quad (3)$$

What is used as the index value is a mean of the weighted brightness Bw(x) in a calculation range 47 where the distance from the center of the image-acquiring catheter 28 ranges inclusively from (R1 - b/2) to the maximum range where an image is acquired. The mean is, for example, an arithmetic mean. The mean may be a geometric mean or a harmonic mean. The control unit 201 calculates the index value related to each scanning line 45. Hereinafter, the mean of the weighted brightness Bw(x) in the calculation range 47 may be referred to as a first index value.

A standard deviation, a variance, and any other statistic of the weighted brightness Bw(x) in the calculation range 47 may also be used as the index value. A multiple or a power of a statistic may also be used as the first index value.

[Modification-1]

Fig. 5B is a view for describing a modification of the method of calculating the index value example-1. In the calculation range 47 in this modification, the distance from the center of the image-acquiring catheter 28 ranges from (R1 - b/2) to (R1 + b), inclusively. The control unit 201 calculates a mean of the weighted brightness Bw(x) in the calculation range 47 and uses the mean as the first index value.

According to this modification, it is possible to calculate and use the first index value which is hardly affected by a region apart from the image-acquiring catheter 28. In a case where the image-acquiring catheter 28 is an IVUS catheter, a scanning target region including a reflector having strong acoustic impedance such as a calcified lesion and a stent may cause multiple reflection or other artifacts to the outside. Making the image-acquiring catheter 28 less susceptible to the region apart from the image-acquiring catheter 28 prevents the image-acquiring catheter 28 from being affected by multiple reflection and other artifacts. Note that the calculation range 47 is not limited to those illustrated in Figs. 5A and 5B.

For example, the calculation range 47 may be a range where the distance from the center of the image-acquiring catheter 28 is (R1 ± b/2), (R1 ± b/4), or (R1 ± b). The calculation range 47 may be a range where the distance from the center of the image-acquiring catheter 28 ranges from R1 to (R1 + b/2) or from (R1 - b/2) to R1, inclusively.

As illustrated above, the calculation range 47 is determined to include a part near the intersection 48 between the scanning line 45 and the representative line 42, thereby calculating an index value which strongly reflects information near the representative line 42 in a tomographic image 41.

[Index Value Example-2]

An index value example calculated with a Rayleigh distribution will be described. The same parts as those in the index value example-1 will be omitted.

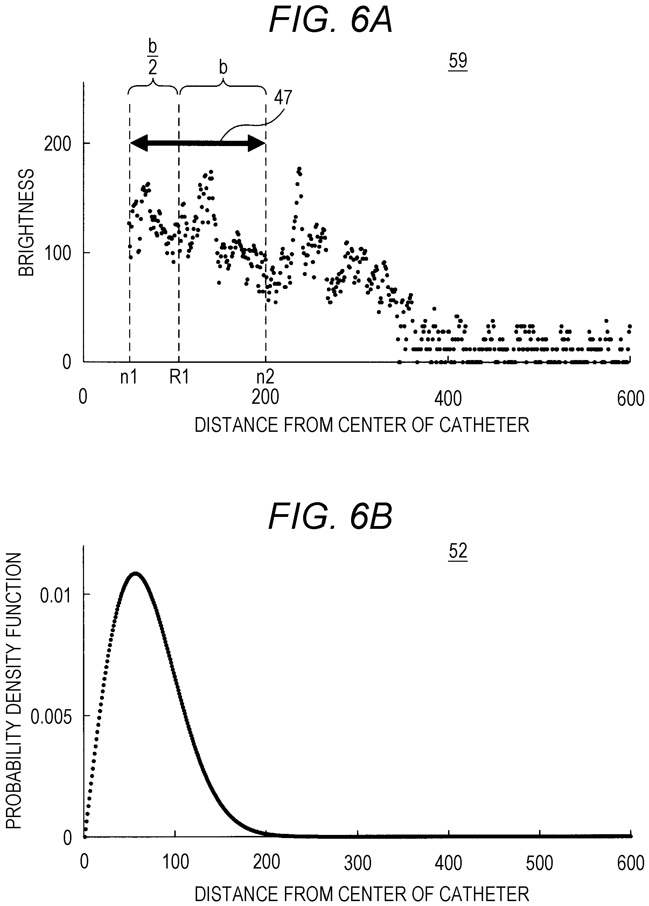

Figs. 6A and 6B are views for describing a method of calculating an index value example-2. Fig. 6A illustrates the brightness graph 59 showing brightness of one scanning line 45. In the brightness graph 59, the distance from the center of the image-acquiring catheter 28 is taken along the abscissa. The brightness is taken along the ordinate in the brightness graph 59. The calculation range 47 is a range where the distance from the center of the image-acquiring catheter 28 ranges from (R1 - b/2) to (R1 + b), inclusively.

Fig. 6B illustrates a Rayleigh distribution graph 52 estimated from brightness data in the calculation range 47. Similarly to the brightness graph 59, the distance from the center of the image-acquiring catheter 28 is taken along the abscissa in the Rayleigh distribution graph 52. The probability density function of the Rayleigh distribution is taken along the ordinate in the Rayleigh distribution graph 52. A probability density function PL(x) of the Rayleigh distribution corresponding to the x-th data from the center of the image-acquiring catheter 28 is calculated by Formula (4).

[Math. 3]

$$PL(x) = \frac{x}{\sigma^2} \exp\left(-\frac{x^2}{2\sigma^2}\right) \quad \cdots\cdots \quad (4)$$

The symbol $\sigma$ is a parameter of the Rayleigh distribution.

An expectation of the Rayleigh distribution represented by Formula (4) is represented by Formula (5), and a variance is represented by Formula (6).

[Math. 4]

$$\text{EXPECTATION} = \sigma\sqrt{\frac{\pi}{2}} \quad \cdots\cdots \quad (5)$$

$$\text{VARIANCE} = \left(2 - \frac{\pi}{2}\right)\sigma^2 \quad \cdots\cdots \quad (6)$$

A maximum likelihood estimate $\sigma g$ of a parameter $\sigma$ is calculated by Formula (7) using the brightness data in the calculation range 47.

[Math. 5]

$$\sigma g = \sqrt{\frac{1}{2n}\sum_{n1}^{n2}(B(x))^2} \quad \cdots\cdots \quad (7)$$

The symbol n is the quantity of the brightness data in the calculation range.

The symbol n1 is the minimum of x in the calculation range.

The symbol n2 is the maximum of x in the calculation range.

The control unit 201 calculates an index value related to each scanning line 45 by Formula (8). Hereinafter, the index value calculated by Formula (8) may be referred to as a second index value.

[Math. 6]

$$\text{INDEX VALUE} = \left(2 - \frac{\pi}{2}\right)\sigma g^2$$

$$= = \frac{1}{2n}\left(2 - \frac{\pi}{2}\right)\sum_{n1}^{n2}(B(x))^2 \quad \cdots\cdots \quad (8)$$

As is clear from Formulae (6) and (8), the second index value represented by Formula (8) is a variance of the Rayleigh distribution calculated based on the maximum likelihood estimate of the parameter $\sigma$. The second index value is not limited to the variance of the Rayleigh distribution.

For example, a standard deviation that is a square root of the variance calculated by Formula (8) may be used as the second index. The expectation of the Rayleigh distribution represented by Formula (5) calculated based on the maximum likelihood estimate of the parameter $\sigma$ may also be used as the second index value. The maximum likelihood estimate $\sigma g$ of the parameter $\sigma$ represented in Formula (7) may also be used as the second index value. A value calculated based on these values may also be used as the second index value.

[Index Value Example-3]

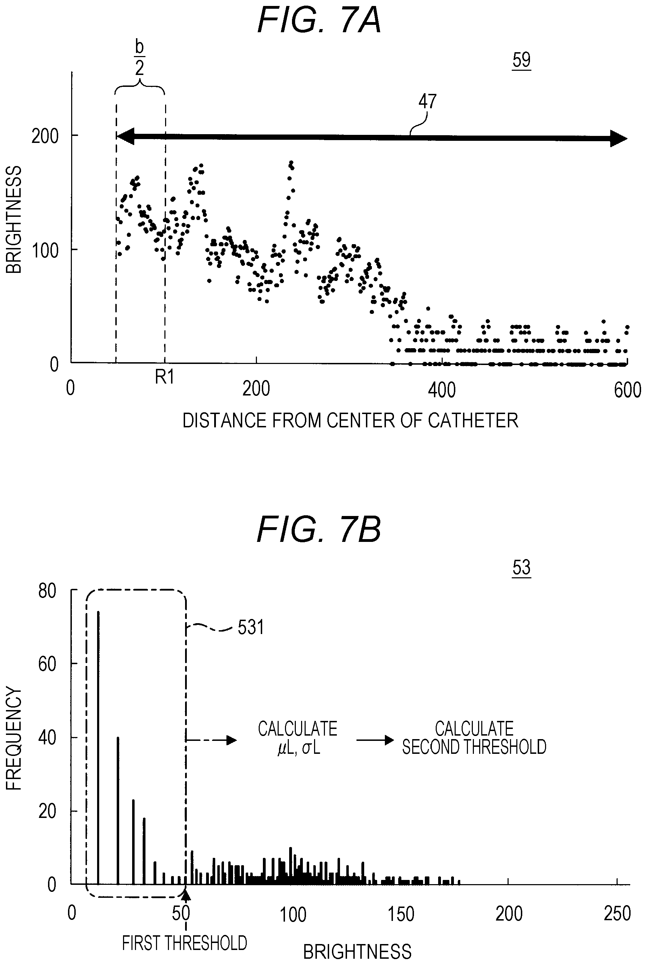

An index value example calculated with a brightness histogram 53 (see Fig. 7B) will be described. The same parts as those in the index value example-1 will be omitted.

Figs. 7A and 7B are views for describing a method of calculating an index value example-3. Fig. 7A illustrates the brightness graph 59 illustrating the brightness of one scanning line 45. In the brightness graph 59, the distance from the center of the image-acquiring catheter 28 is taken along the abscissa. The brightness is taken along the ordinate in the brightness graph 59. In the calculation range 47, the distance from the center of the image-acquiring catheter 28 ranges inclusively from (R1 - b/2) to the maximum range where an image is acquired.

Fig. 7B illustrates the brightness histogram 53 calculated based on brightness data in the calculation range 47. The brightness is taken along the abscissa in the brightness histogram 53. The frequency is taken along the ordinate in the brightness histogram 53. In the following description, a part of the brightness histogram 53 where the brightness is less than a predetermined first threshold is referred to as a low brightness region 531. The first threshold is, for example, 64.

From the brightness data in the calculation range 47, the control unit 201 extracts data in which the brightness is less than the first threshold. The control unit 201 calculates a mean $\mu L$ and a standard deviation $\sigma L$ of the extracted data. Based on Formula (9), the control unit 201 calculates a second threshold, a threshold on the high brightness side.

$$\text{Second threshold} = \mu L + 3\sigma L \quad \cdots\cdots \quad (9)$$

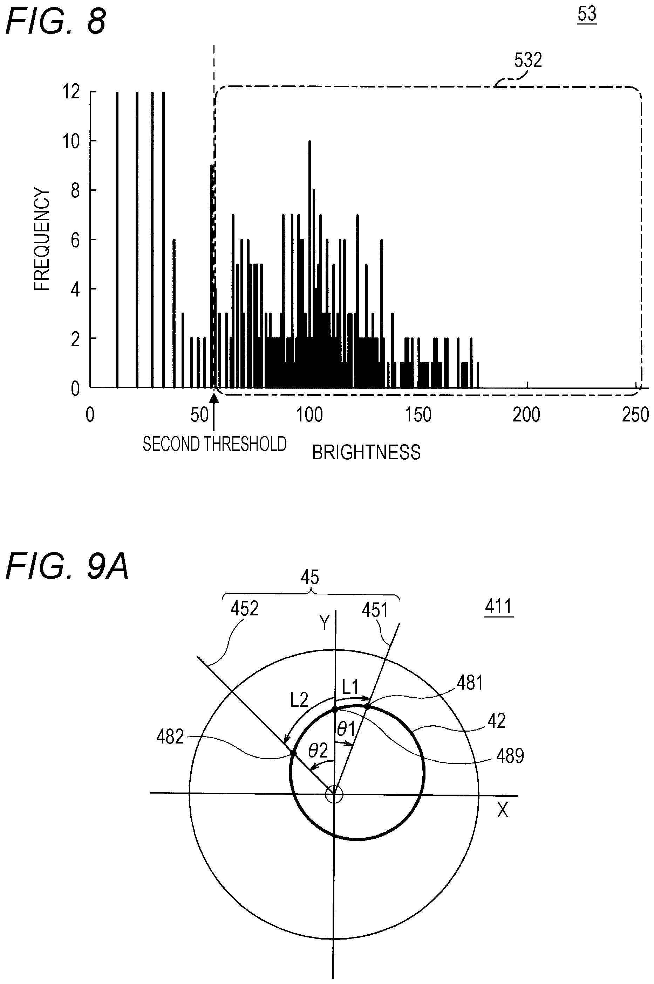

Fig. 8 is a view for describing the method of calculating the index value example-3. Similarly to Fig. 7B, Fig. 8 illustrates the brightness histogram 53 calculated based on the brightness data in the calculation range 47. In Fig. 8, the ordinate is enlarged to the range of 0 to 12. The second threshold calculated by Formula (9) is taken along the abscissa in Fig. 8. In the following description, a part of the brightness histogram 53 where the brightness is equal to or more than the predetermined second threshold is referred to as a high brightness region 532.

From the brightness data in the calculation range 47, the control unit 201 extracts data included in the high brightness region 532. The control unit 201 calculates a standard deviation $\sigma H$ of the extracted data. The standard deviation $\sigma H$ is an example of a second statistic. Hereinafter, the standard deviation $\sigma H$ may be referred to as a third index value. The third index value is not limited to $\sigma H$.

For example, a variance of data included in the high brightness region 532 may be used as the third index value. Any statistic such as an arithmetic mean, a geometric mean, a harmonic mean, a median, and a mode value of the data included in the high brightness region 532 may also be used as the third index value.

The calculation formula of the second threshold is not limited to Formula (9). The second threshold may be a predetermined constant. The calculation range 47 is not limited to a range where the distance from the center of the image-acquiring catheter 28 ranges inclusively from (R1 - b/2) to the maximum range where an image is acquired. For example, the calculation range 47 may be a range where the distance from the center of the image-acquiring catheter 28 is (R1 - b/2) or more, (R1 + b), (R1 ± b/2), (R1 ± b/4), or (R1 ± b). The calculation range 47 may be a range where the distance from the center of the image-acquiring catheter 28 ranges from R1 to (R1 + b/2) or from (R1 - b/2) to R1, inclusively.

The calculation range 47 for calculating the first index value, the second index value, and the third index value may be common or different.

The index values may be calculated based on data at one phase during the conversion of a signal acquired by a sensor 282 (see Fig. 25) into brightness data. The index values may be calculated based on the signal itself acquired by the sensor 282.

For example, any one of the first index value, the second use value, and the third index value can be selected and used as an index value of each scanning line 45. This case generates a black-to-white gradient index value stripe 43 where the index values are allocated to the gray scale from black to white, which determines the index value image 44 as a monochrome image.

The three index values, the first index value, the second use value, and the third index value, may be used simultaneously. For example, the control unit 201 normalizes each of the first index value, the second use value, and the third index value to an integer from 0 to 255. The control unit 201, for example, allocates the normalized index values to the brightness of red (R), green (G), and blue (B) in any order without overlaps. Any combination of an index value number and a color is employable.

The allocation to the brightness of RGB in this manner generates an index value stripe 43 where the index values are colored with what is called the full 24-bit color, which determines the index value image 44 as a full-color image.

RGB is an example of a color channel constituting a color image displayed on the display unit 205. Instead of RGB, the control unit 201 may allocate each index value to a channel corresponding to any color space such as an HLS color space and HSV (hue, saturation, value) color space.

Adding an alpha channel to RGB, the control unit 201 may calculate four index values and allocate the four index values to RGBA. The control unit 201 may also allocate index values to four channels consisting of cyan, magenta, yellow, and black (CYMK).

Figs. 9A to 9C are views for describing a method of converting between an angle and a length. Fig. 9A schematically illustrates an image in which the representative line 42 is superimposed on the X-Y format tomographic image 411. Among scanning lines 45 forming the X-Y format tomographic image 411, Fig. 9A illustrates two scanning lines 45, a first scanning line 451 and a second scanning line 452. In the X-Y format tomographic image 411, the intersection 48 between a reference line extending upward from the center of the image-acquiring catheter 28 and the representative line 42 is denoted by a reference intersection 489. The reference intersection 489 is an example of a reference point determined on the representative line 42.

The first scanning line 451 is positioned clockwise from the reference line at an angle of $\theta 1$. The intersection 48 between the first scanning line 451 and the representative line 42 is denoted by a first intersection 481. A length from the reference intersection 489 to the first intersection 481 along the representative line 42 is denoted by L1.

Similarly, the second scanning line 452 is positioned counterclockwise from the reference line at an angle of $\theta 2$. The intersection 48 between the second scanning line 452 and the representative line 42 is denoted by a second intersection 482. A length from the reference intersection 489 to the second intersection 482 along the representative line 42 is denoted by L2.

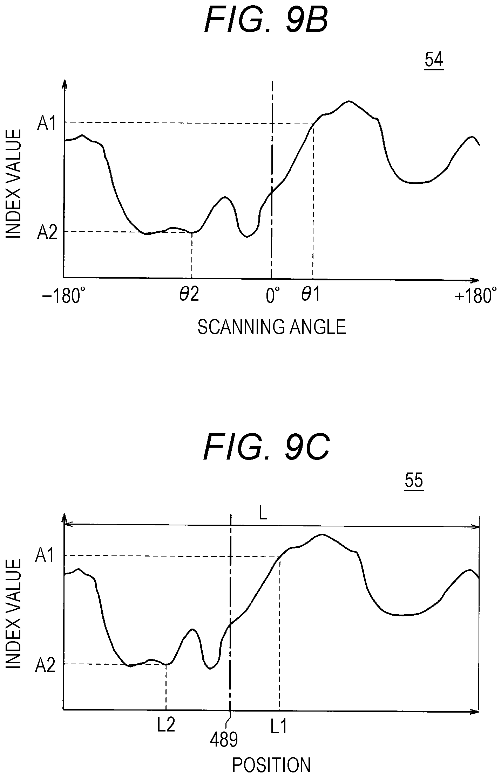

Fig. 9B illustrates the angle-index value graph 54. A1 denotes the index value corresponding to the first scanning line 451 and A2 denotes the index value corresponding to the second scanning line 452. Fig. 9C illustrates the position-index value graph 55. In Fig. 9A, the center of the representative line 42 is on the upper right position relative to the center of the image-acquiring catheter 28, and a position on the abscissa of the position-index value graph 55 corresponding to the reference intersection 489 is closer to the left side than the center of the abscissa. In the position-index value graph 55, a distance between the minimum and the maximum on the abscissa is the length L of the index value stripe 43.

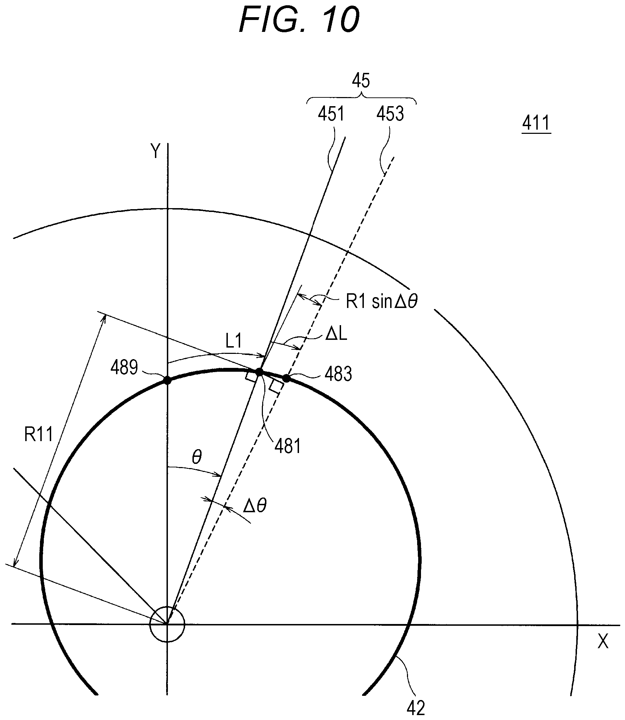

Fig. 10 is a view for describing the method of converting between an angle and a length. With reference to Fig. 10, hereinafter described is a method of converting the angle-index value graph 54 illustrated in Fig. 9B into the position-index value graph 55 illustrated in Fig. 9C.

Fig. 10 illustrates an enlarged upper right part of Fig. 9A. A distance between the center of the image-acquiring catheter 28 and the first intersection 481 is denoted by R11. A third scanning line 453 indicated by a broken line is a scanning line 45 adjacent to the first scanning line 451. The intersection 48 between the third scanning line 453 and the representative line 42 is denoted by a third intersection 483.

An angle between the first scanning line 451 and the third scanning line 453 is indicated by $\Delta\theta$. In all the scanning lines 45 forming the X-Y format tomographic image 411, $\Delta\theta$ is constant. For example, when one tomographic image 41 includes five hundred and twelve scanning lines 45, $\Delta\theta$ is about 0.7 degrees. In Fig. 10, $\Delta\theta$ is schematically illustrated at a large angle. A length from the first intersection 481 to the third intersection 483 along the representative line 42 is indicated by $\Delta L$.

The angle $\Delta\theta$ is small, and $\Delta L$ can be approximated by Formula (10).

$$\Delta L = R11 \times \sin\Delta\theta \quad \cdots\cdots \quad (10)$$

Similarly, an arc length $\Delta Ln$ between the n-th scanning line 45 and the (n + 1)th scanning line 45 can be approximated by Formula (11).

$$\Delta Ln = R1n \times \sin\Delta\theta \quad \cdots\cdots \quad (11)$$

R1n is a distance between the center of the image-acquiring catheter 28 and the intersection 48 between the n-th scanning line 45 and the representative line 42.

The length L1 along the representative line 42 can be calculated by adding $\Delta L$ represented by Formula (10) while sequentially moving the scanning line 45 from the reference line to the first scanning line 451. Through these steps, the control unit 201 can generate the position-index value graph 55 based on the angle-index value graph 54. The control unit 201 converts index values on the ordinate of the position-index value graph 55 into colors as described above, thereby generating one index value stripe 43 corresponding to one tomographic image 41.

[Representative Line Determination-1]

The control unit 201 generates, for example, a circle that passes through three points designated by the user on the X-Y format tomographic image 411 and uses the circle as the representative line 42. The control unit 201 may superimpose a template indicating a circle on the X-Y format tomographic image 411, receive an input from the user to change the position and radius, and use the circle determined by the user as the representative line 42. Alternatively, the control unit 201 may receive an input from the user to designate the representative line 42 through any user interface.

[Representative Line Determination-2]

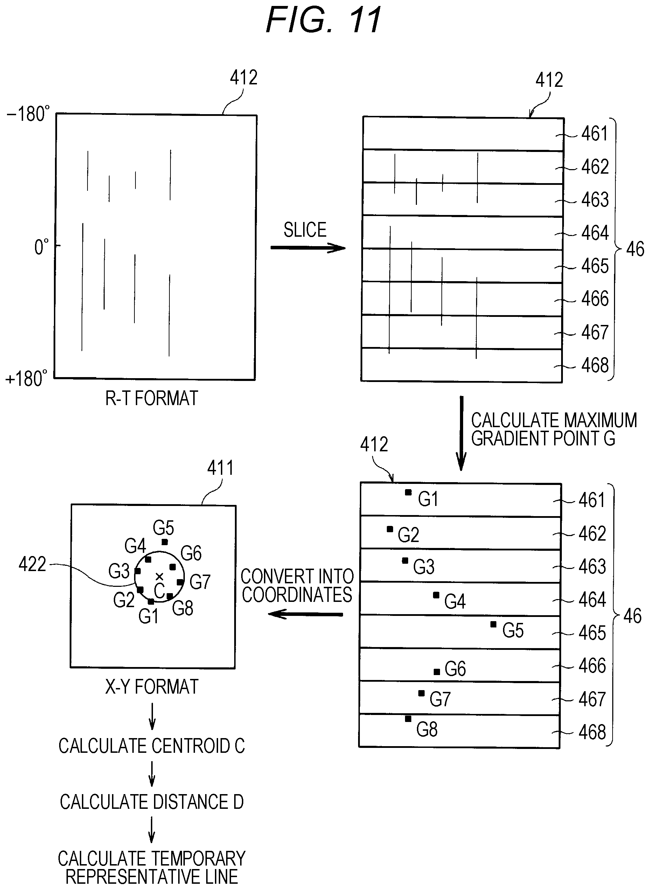

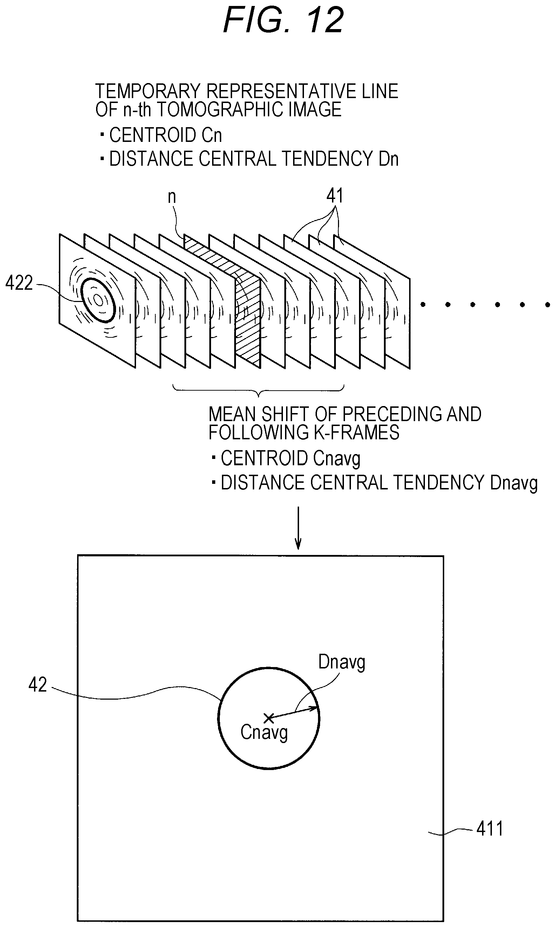

Figs. 11 and 12 are views for describing a method of determining the representative line 42. With reference to Figs. 11 and 12, hereinafter described is an example of how the control unit 201 automatically determines the representative line 42.

The control unit 201 slices the R-T format tomographic image 412 into, for example, twenty-five blocks 46 by a line parallel to R-axis. When one R-T format tomographic image 412 is formed by five hundred and twelve scanning lines 45, one block 46 includes twenty or twenty-one scanning lines 45.

Moving on to Fig. 12, a relation between a plurality of tomographic images 41 acquired by three-dimensional scanning will be described. The hatching indicates an in-process tomographic image 41. The control unit 201 processes a group of tomographic images 41 including the in-process tomographic image 41 combined with preceding and following K-frames of tomographic images 41. In the following description, K is 3, and the control unit 201 combines and processes a total of seven tomographic images 41 from the (n - 3)th tomographic image 41 to the (n + 3)th tomographic image 41.

Fig. 11 schematically illustrates a case where the number of blocks 46 is eight. From the scanning angle of - 180 degrees, the first block to the eighth block are denoted in order by reference numerals 461 to 468. The control unit 201 calculates a maximum gradient point G for each block 46. The maximum gradient point G is a position showing the largest change in direction in which the brightness increases in R-direction, that is, the largest gradient.

The gradient of brightness at each point on the R-T format tomographic image 412 corresponding to z-coordinate can be calculated by Formula (12). Formula (12) is an expression representing the convolution in which a Sobel operator F is applied to a flat surface obtained by cutting, at a scanning angle of $\theta$, a plurality of R-T format tomographic images 412 acquired by three-dimensional scanning.

[Math. 7]

$$g(r, \theta, z)$$

$$= \sum_{j=-w}^{j=w} \sum_{k=-w}^{k=w} F(k, j) I(r+k, \theta, z+j) \quad \cdots\cdots \quad (12)$$

The symbols $(r, \theta, z)$ are coordinates in the R-T format tomographic image corresponding to the z-coordinate.

The symbol r is a position of the distance from the center of the image-acquiring catheter 28.

The symbol $\theta$ is a position of the scanning angle.

The symbol z is a coordinate in the axial direction of the image-acquiring catheter 28 during the three-dimensional scanning.

The symbols $g(r, \theta, z)$ are gradients of brightness at coordinates $(r, \theta, z)$.

The symbol $I(r, \theta, z)$ are the brightness of the R-T format tomographic image corresponding to the $(r, \theta, z)$ coordinates z.

The symbol F(k, j) is (k, j) elements of the Sobel operator F.

The symbols j, k, and w are parameters of the convolution.

The Sobel operator F used in Formula (12) is an operator used for contour detection in the horizontal direction. An example of the Sobel operator used when combining seven tomographic images 41 as described above is represented by Formula (13). The elements of the j-th row and the k-th column of Formula (13) are represented by F(k, j) in Formula (12).

[Math. 8]

$$
F = \begin{bmatrix}
-130 & -120 & -78 & 0 & 78 & 120 & 130 \\
-180 & -195 & -156 & 0 & 156 & 195 & 180 \\
-234 & -312 & -390 & 0 & 390 & 312 & 234 \\
-260 & -390 & -780 & 0 & 780 & 390 & 260 \\
-234 & -312 & -390 & 0 & 390 & 312 & 234 \\
-180 & -195 & -156 & 0 & 156 & 195 & 180 \\
-130 & -120 & -78 & 0 & 78 & 120 & 130
\end{bmatrix}
$$

$$\cdots\cdots \quad (13)$$

When using the $7 \times 7$ Sobel operator F represented by Formula (13), w in Formula (12) is 3, which is half the kernel width of the Sobel operator F. Note that the Sobel operator F is not limited to Formula (13). It is possible to use any Sobel operator F that enables contour detection in the horizontal direction. The Sobel operator F used for combing the preceding and following K-frames of tomographic images 41 is a square matrix with the vertical line of (2K + 1) and the horizontal line of (2K + 1).

With regard to the R-T format tomographic image 412 corresponding to each z-coordinate, the control unit 201 extracts the coordinates (r, θ, z) in which g(r, θ, z) calculated by Formula (12) for each block 46 is the largest. The extracted coordinates (r, θ, z) are maximum gradient points G of the blocks 46. Fig. 11 schematically illustrates the maximum gradient points G extracted from the blocks 46, that is, G1 to G8 extracted from the first block 461 to the eighth block 468, on the R-T format tomographic image 412.

The control unit 201 can convert the R-T format tomographic image 412 into the X-Y format tomographic image 411 by known coordinate conversion. In Fig. 11, the maximum gradient point G1 to the maximum gradient point G8 are arranged in a substantially circumferential shape on the X-Y format tomographic image 411.

With regard to the maximum gradient point G1 to the maximum gradient point G8 on the X-Y format tomographic image 411, the control unit 201 calculates coordinates of a centroid C. A method of calculating coordinates of the centroid C from coordinates of a plurality of planar points is known, and the details thereof will be omitted. The control unit 201 calculates distances D between the calculated centroid C and the maximum gradient points G. The control unit 201 calculates a median Dc which is a central tendency of the calculated distances D.

Through these steps, it is possible to determine a temporary representative line 422 whose center is the centroid C of the maximum gradient points G and whose radius is the median Dc of the distances D between the centroid C and the maximum gradient points G. A mean may be used as the central tendency of the distances D.

Note that positions of the extracted maximum gradient points G may be inappropriate due to noise, artifacts, and the like in a tomographic images 41. In order to avoid adverse effects caused by the mixture of the inappropriate maximum gradient points G, it is desirable that the control unit 201 excludes outliers of the maximum gradient points G.

For example, with regard to the second and subsequent tomographic images 41, the control unit 201 calculates distances D between the centroid C and the maximum gradient points G, and then, excludes a distance D in which a difference from a central tendency Dc of distances D in the previous tomographic image 41 exceeds a predetermined threshold. After that, the control unit 201 calculates a central tendency Dc of the remaining distances D. Through these steps, the control unit 201 determines the temporary representative line 422 that is hardly affected by the inappropriate maximum gradient points G.

Moving on to Fig. 12, hereinafter described is processing after determining the temporary representative line 422 for each tomographic image 41. In the following description, the center of the temporary representative line 422 in the n-th tomographic image 41 is referred to as Cn, and the radius of the temporary representative line 422 is referred to as Dn.

With regard to the n-th tomographic image 41, the control unit 201 calculates a moving average of coordinates of the center C and a moving average of the radius D in each of the preceding and following K-frames of tomographic images 41. For example, when K is 3, the control unit 201 calculates a mean Cnavg of coordinates of the center C and a mean Dnavg of the radius D for the temporary representative line 422 for a total of seven tomographic images 41, that is, from the (n - 3)th tomographic image 41 to (n + 3)th tomographic image 41. The control unit 201 uses the calculated Cnavg and Dnavg as the center and radius of the representative line 42 in the n-th tomographic image 41.

The above description has illustrated a case where the same number of tomographic images 41 are used at the time of calculating gradients described with reference to Formulae (12) and (13) and at the time of calculating Cnavg and Dnavg. However, different numbers of tomographic images 41 may be used for those calculations. For example, the control unit 201 may calculate gradients based on K = 3 and calculate Cnavg and Dnavg based on K = 7.

With regard to tomographic images 41 near both ends of one batch of tomographic images 41, for example, the center and the radius of the temporary representative line 422 are used as the center and the radius of the representative line 42. For tomographic images 41 near both ends of the batch of tomographic images 41, the number of tomographic images 41 up to the very end may be used as the value of K.

These steps creates the representative line 42 that forms a three-dimensional shape smoothly connected in a three-dimensional space formed by the batch of tomographic images 41 while avoiding the influence of noise, artifacts, and the like in the tomographic images 41.

The methods of determining the representative line 42 described in Representative Line Determination-1 and Representative Line Determination-2 are examples, and the determination methods are not limited thereto. For example, the representative line 42 may be determined on the X-Y format tomographic image 411 by pattern matching or the like.

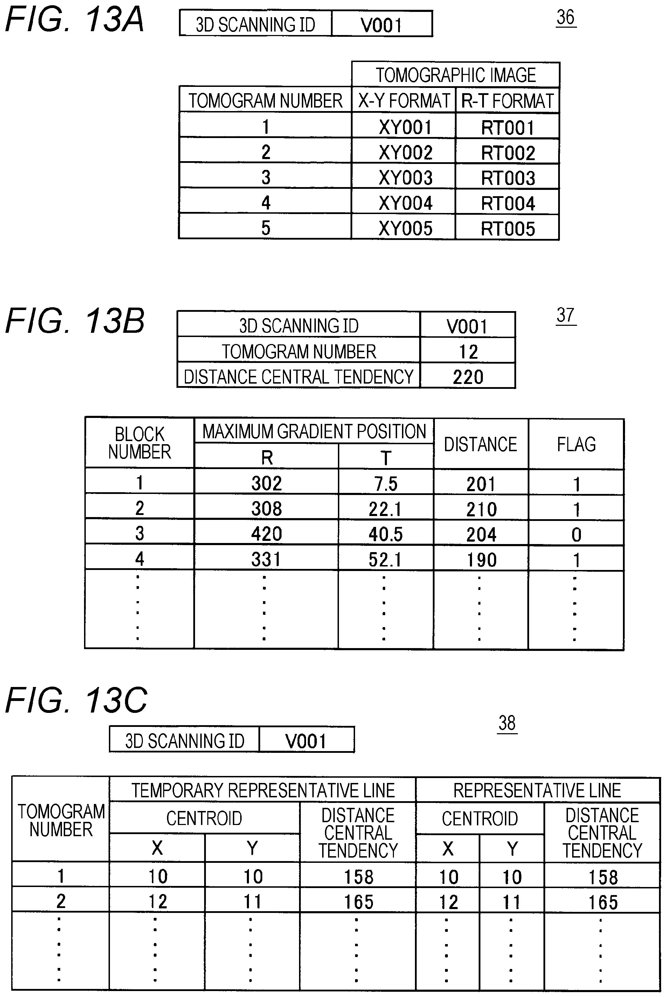

Fig. 13A is a view for describing a record layout of the tomographic image DB 36. The tomographic image DB 36 is a database for recording tomographic images 41 created by three-dimensional scanning. The tomographic image DB 36 includes a 3D scan ID field, a tomogram number field, and a tomographic image field. The tomographic image field has an X-Y format field and an R-T format field.

The 3D scan ID field is for recording a 3D scan ID assigned for each three-dimensional scanning. The tomogram number field is for recording numbers indicating sequences of the tomographic images 41 created by one three-dimensional scanning. The X-Y format field is for recording X-Y format tomographic images 411. The R-T format field is for recording R-T format tomographic images 412. The tomographic image DB 36 has one record for one tomographic image 41.

Note that only the R-T format tomographic images 412 may be recorded in the tomographic image DB 36, and the control unit 201 may create X-Y format tomographic images 411 by coordinate conversion as necessary. Instead of using the tomographic image DB 36 for recording tomographic images 41, the control unit 201 may create tomographic images 41 on an as-needed basis based on a database in which sound ray data or the like at a stage before creating the tomographic images 41 is recorded.

Instead of using the tomogram number field, the tomographic image DB 36 may have a field for recording a distance from the starting point of three-dimensional scanning to a position where each tomographic image 41 is acquired. For example, from an MDU 289 (see Fig. 25), the control unit 211 can acquire the distance from the starting point of three-dimensional scanning to the position where each tomographic image 41 is acquired.

Fig. 13B is a view for describing a record layout of the maximum gradient point DB 37. The maximum gradient point DB 37 is a database for recording information on maximum gradient points G described with reference to Fig. 11. The maximum gradient point DB 37 has the 3D scan ID field, the tomogram number field, a distance central tendency field, a block number field, a maximum gradient position field, a distance field, and a flag field. The maximum gradient position field has an R field and a T field.

The 3D scan ID field is for recording a 3D scan ID assigned for each three-dimensional scanning. The tomogram number field is for recording numbers indicating sequences of the tomographic images 41 created by one three-dimensional scanning. The distance central tendency field is for recording a central tendency of the distances D between the centroid C and the maximum gradient points G described with reference to Fig. 11.

The block number field is for recording numbers of the blocks 46. The R field is for recording R-coordinates of the maximum gradient points G in the blocks 46, that is, distances from the center of the image-acquiring catheter 28. The T field is for recording T-coordinates of the maximum gradient points G in the blocks 46, that is, scanning angles.

The distance field is for recording distances D between the centroid C and the maximum gradient positions G. The flag field is for recording a flag indicating whether each maximum gradient position G is an outlier. A maximum gradient position G with the flag '1' is not an outlier but is used for calculating a central tendency to be recorded in the distance central tendency field. A maximum gradient position G with the flag '0' is an outlier and is not used for calculating a central tendency to be recorded in the distance central tendency field. The maximum gradient point DB 37 has one record for one block 46.

Fig. 13C is a view for describing a record layout of the representative line DB 38. The representative line DB 38 is a database for recording information on each representative line 42. The representative line DB 38 includes the 3D scan ID field, the tomogram number field, a temporary representative line field, and a representative line field. The temporary representative line field and the representative line field each have a centroid field and a distance central tendency field. The centroid field of the temporary representative line field and that of the representative line field both have an X field and a Y field.

The 3D scan ID field is for recording a 3D scan ID assigned for each three-dimensional scanning. The tomogram number field is for recording numbers indicating sequences of the tomographic images 41 created by one three-dimensional scanning. X-coordinate and Y-coordinate of the centroid C of the maximum gradient points G described with reference to Fig. 11 are recorded in the centroid field of the temporary representative line field. In the distance central tendency field of the temporary representative line field, the central tendency $D_c$ of the distances D is recorded.

In the centroid field of the representative line field, a moving average of the X-coordinate and the Y-coordinate of the centroid $C_{navg}$, that is, a moving average of the centroid C described with reference to Fig. 12, is recorded. In the distance central tendency field of the representative line field, a distance central tendency $D_{navg}$, or a moving average of the distance central tendency D described with reference to Fig. 12, is recorded. The representative line DB 38 has one record for one tomographic image 41.

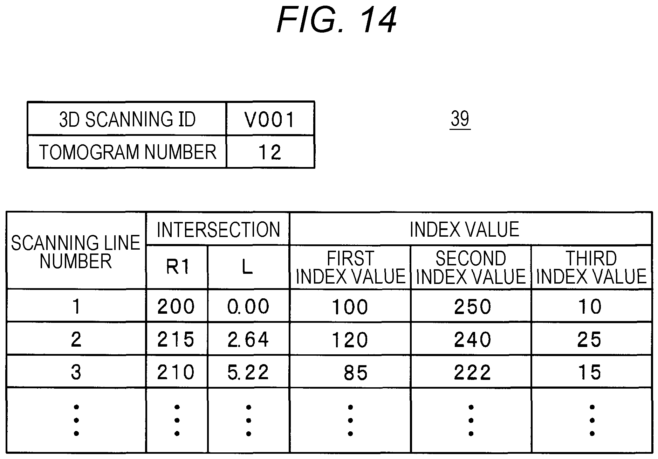

Fig. 14 is a view for describing a record layout of the index value DB 39. The index value DB39 is a database for recording index values corresponding to respective scanning lines 45. The index value DB 39 includes the 3D scan ID field, the tomogram number field, a scanning line number field, an intersection field, and an index value field.

The intersection field has an R1 field and an L field. The index value field has a first index value field, a second index value field, and a third index value field. Note that the index value field does not necessarily have a subfield. The index value field may have four or more subfields.

The 3D scan ID field is for recording a 3D scan ID assigned for each three-dimensional scanning. The tomogram number field is for recording numbers indicating sequences of the tomographic images 41 created by one three-dimensional scanning. The scanning line number field is for recording numbers of the scanning lines. The R1 field is for recording R1, that is, a distance between the center of the image-acquiring catheter 28 and the intersection 48. The L field is for recording a length L from the reference intersection 489 to the intersection 48 along each representative line 42. The first index value field, the second index value field, and the third index value field are for recording a first index value, a second index value, and a third index value, respectively.

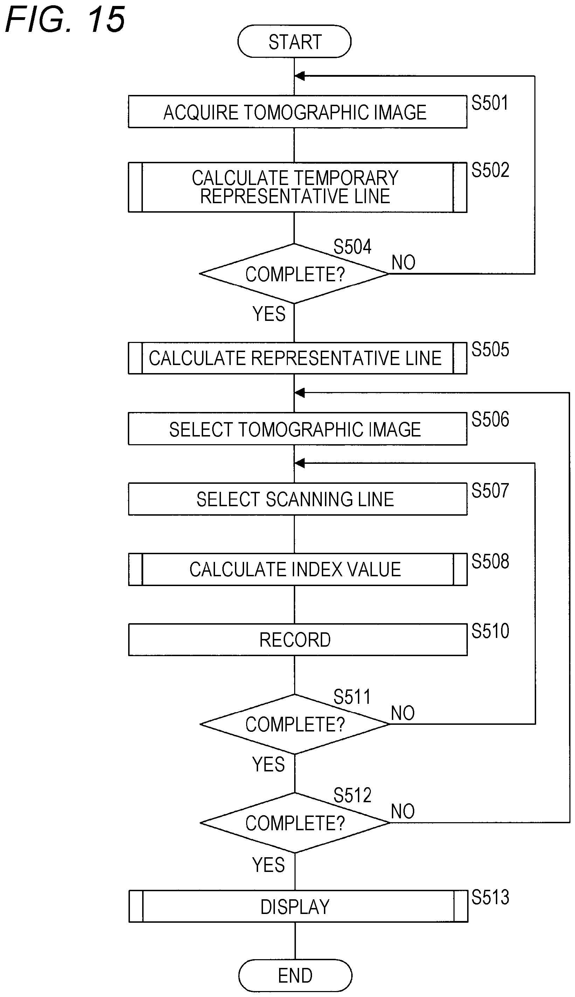

Fig. 15 is a flowchart for describing a processing flow of a program. The control unit 201 acquires one tomographic image 41 from the tomographic image DB 36 (Step S501). The control unit 201 invokes a subroutine for temporary representative line calculation (Step S502). The subroutine for temporary representative line calculation is a subroutine for calculating the center and the radius of the temporary representative line 422 described with reference to Fig. 11. A processing flow of the subroutine for temporary representative line calculation will be described later.

The control unit 201 determines whether the processing of the tomographic images 41 included in one batch of tomographic images 41 has been completed (Step S504). Determining that the processing has not been completed (NO in Step S504), the control unit 201 returns to Step S501. Determining that the processing has been completed (YES in Step S504), the control unit 201 invokes a subroutine for representative line calculation (Step S505). The subroutine for representative line calculation is a subroutine for calculating the center and the radius of the representative line 42 based on a moving average of the temporary representative line 422 as described with reference to Fig. 12. A processing flow of the subroutine for representative line calculation will be described later.

The control unit 201 selects a tomographic image 41 whose index value is to be calculated (Step S506). The tomographic image 41 selected in Step S506 is one of the tomographic images 41 acquired in the loop from Step S501 to Step S504.

The control unit 201 selects one of the scanning lines 45 forming the tomographic image 41 selected in Step S506 (Step S507). Each time Step S507 is executed, the control unit 201 selects one scanning line 45 clockwise from the reference intersection 489 described with reference to Figs. 9A and 10.

The control unit 201 invokes a subroutine for index value calculation (Step S508). The subroutine for index value calculation is a subroutine for calculating an index value based on one scanning line 45. A processing flow of the subroutine for index value calculation will be described later.

The control unit 201 extracts a record of the index value DB39 which corresponds to the scanning line 45 selected in Step S507. In the R1 field, the L field, and the index value field, the control unit 201 respectively records a distance R1, a length L, and an index value calculated by the subroutine for index value calculation (Step S510).

The control unit 201 determines whether the processing of the scanning lines 45 forming the tomographic image 41 acquired in Step S506 is completed (Step S511). Determining that the processing has not been completed (NO in Step S511), the control unit 201 returns to Step S507. Determining that the processing has been completed (YES in Step S511), the control unit 201 determines whether the processing of the batch of tomographic images 41 has been completed (Step S512).

Determining that the processing has not been completed (NO in Step S512), the control unit 201 returns to Step S506. Determining that the processing has been completed (YES in Step S512), the control unit 201 invokes a subroutine for display (Step S513). The subroutine for display is a subroutine for displaying the index value image 44 based on the index value DB 39. A processing flow of the subroutine for display will be described later. After that, the control unit 201 ends the processing.

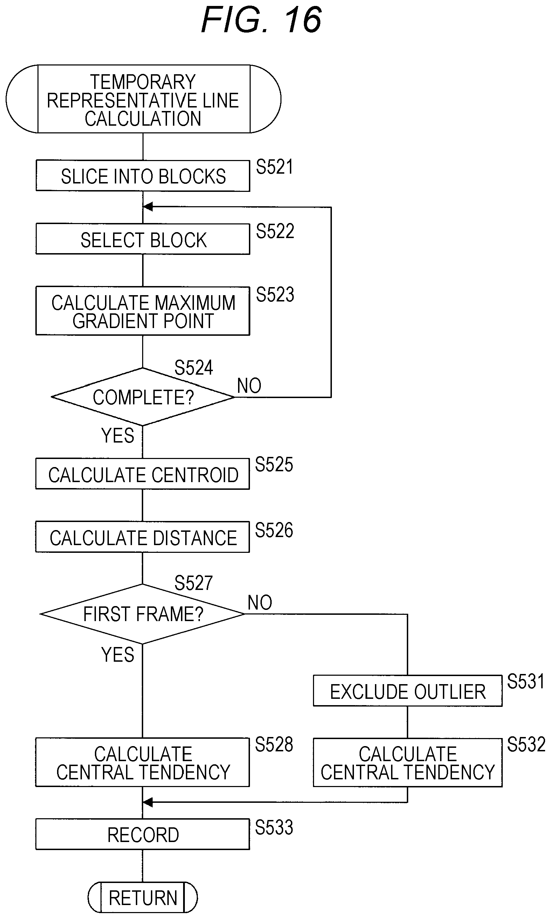

Fig. 16 is a flowchart for describing the processing flow of the subroutine for temporary representative line calculation. The subroutine for temporary representative line calculation is a subroutine for calculating the center and the radius of the temporary representative line 422 described with reference to Fig. 11 based on one tomographic image 41.

As described with reference to Fig. 11, the control unit 201 slices the in-process tomographic image 41 into a predetermined number of blocks 46 (Step S521). The control unit 201 selects one block 46 (Step S522). The control unit 201 calculates coordinates of maximum gradient points G in the block 46 (Step S523). Specifically, the control unit 201 calculates a gradient of brightness at each position in the block 46 based on Formula (12). The control unit 201 extracts coordinates of the position showing the largest calculated gradient of brightness.

The control unit 201 determines whether the processing of the blocks 46 sliced in Step S521 has been completed (Step S524). Determining that the processing has not been completed (NO in Step S524), the control unit 201 returns to Step S522.

Determining that the processing has been completed (YES in Step S524), the control unit 201 calculates coordinates of a centroid C of a plurality of maximum gradient points G (Step S525). The control unit 201 calculates distances D between the maximum gradient points and the centroid C (Step S526) .

The control unit 201 determines whether the in-process tomographic image 41 is the first frame of the batch of tomographic images 41 (Step S527). Determining that the in-process image is the first frame (YES in Step S527), the control unit 201 calculates a central tendency Dc of the distances D calculated in Step S526 (Step S528). The central tendency Dc is, for example, a median.

Determining that the in-process image is not the first frame (NO in Step S527), the control unit 201 excludes an outlier from the distances D calculated in Step S526 (Step S531). The outlier is, for example, a distance D at which a difference from the central tendency Dc calculated in the previous tomographic image 41 exceeds a predetermined threshold. The control unit 201 calculates a central tendency Dc of the distances D (Step S532) .

On completion of Step S528 or Step S532, the control unit 201 records, in the representative line DB 38, the centroid C calculated in Step S525 and the central tendency Dc calculated in Step S528 or Step S532 (Step S533).

Specifically, the control unit 201 extracts a record in the representative line DB 38 which corresponds to the in-process tomographic image 41. The control unit 201 records X-coordinate and Y-coordinate of the centroid C calculated in Step S525 in the centroid field of the temporary representative line field of the extracted record. The control unit 201 records the central tendency Dc calculated in Step S528 or Step S532 in the central tendency field of the temporary representative line field of the extracted record. After that, the control unit 201 ends the processing.

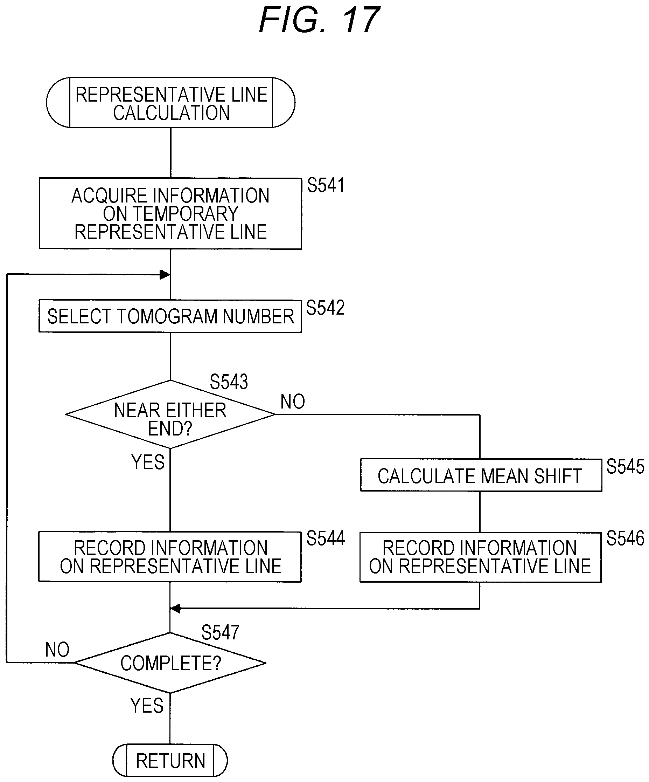

Fig. 17 is a flowchart for describing the processing flow of the subroutine for representative line calculation. From the representative line DB 38, the control unit 201 acquires information on the batch of tomographic images 41 recorded in the temporary representative line field (Step S541). The control unit 201 selects a tomogram number of the tomographic image 41 to be processed (Step S542). For example, each time Step S542 is executed, the control unit 201 sequentially increments the tomogram number by 1 from 1.

The control unit 201 determines whether the tomogram number selected in Step S542 indicates an image near either end of the batch of tomographic images 41 (Step S543). Specifically, as described with reference to Fig. 12, in a case where a moving average of the preceding and following K-frames of tomographic images 41 is used, when the tomographic image number selected in Step S542 is equal to or less than K and equal to or more than (the total number of the tomographic images 41 - K), the control unit 201 determines that the selected tomographic image number indicates an image near either end.

Determining that the tomogram number indicates an image near either end (YES in Step S543), the control unit 201 records the same information in the representative line field as the information recorded in the temporary representative line field (Step S544). Determining that the tomogram number does not indicate an image near either end (NO in Step S543), the control unit 201 calculates a moving average of the centroid C and that of the central tendency Dc using data of a predetermined number of tomogram numbers preceding and following the in-process tomogram number (Step S545).

The control unit 201 records the calculated moving average in the representative line field of the representative line DB 38 (Step S546). Specifically, the control unit 201 extracts a record in the representative line DB 38 which corresponds to the in-process tomographic image 41. The control unit 201 records the moving average of the centroid C calculated in Step S545 in the centroid field of the representative line field of the extracted record. The control unit 201 records the moving average of the central tendency Dc calculated in Step S545 in the central tendency field of the representative line field of the extracted record.

The control unit 201 determines whether the processing of the temporary representative line 422 acquired in Step S541 is completed (Step S547). Determining that the processing has not been completed (NO in Step S547), the control unit 201 returns to Step S542. Determining that the processing has been completed (YES in Step S547), the control unit 201 ends the processing.

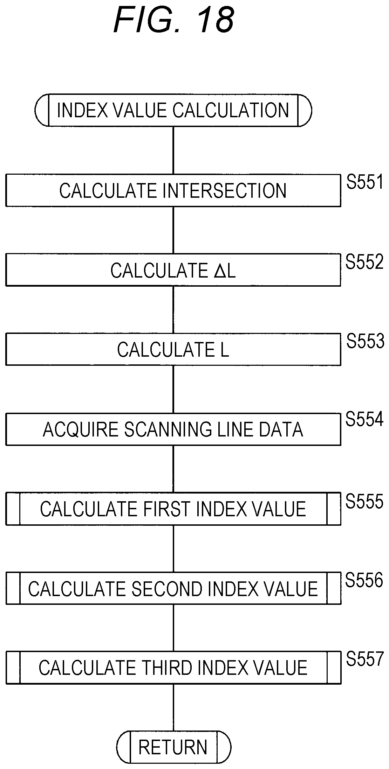

Fig. 18 is a flowchart for describing the processing flow of the subroutine for index value calculation. The subroutine for index value calculation is a subroutine for calculating an index value based on one scanning line 45.

The control unit 201 calculates coordinates of the intersection 48 between the in-process scanning line 45 and the representative line 42 (Step S551). Herein, the control unit 201 calculates coordinates of the intersection 48 in both X-Y and R-T coordinate systems. Based on Formula (10), the control unit 201 calculates $\Delta L$, an arc length from the intersection 48 on the previous scanning line 45 (Step S552).

The control unit 201 calculates an arc length L from the reference intersection 489, that is, a sum of $\Delta L$ calculated in the past (Step S553). With regard to a scanning line 45 having a scanning angle of 180 degrees or more, the control unit 201 subtracts a sum of $\Delta L$ from the circumferential length of the representative line 42 to calculate an arc length L. Through Step S553, the abscissa of the angle-index value graph 54 described with reference to Fig. 3 is converted, thereby calculating the position-index value graph 55.

The control unit 201 acquires scanning line data related to the in-process scanning line 45 (Step S554). The control unit 201 invokes a subroutine for first index value calculation (Step S555). The subroutine for first index value calculation is a subroutine for calculating a first index value described with reference to Figs. 4A, 4B, and 5A. A processing flow of the subroutine for first index value calculation will be described later.

The control unit 201 invokes a subroutine for second index value calculation (Step S556). The subroutine for second index value calculation is a subroutine for calculating a second index value described with reference to Figs. 6A and 6B. A processing flow of the subroutine for second index value calculation will be described later.

The control unit 201 invokes a subroutine for third index value calculation (Step S557). The subroutine for third index value calculation is a subroutine for calculating a third index value described with reference to Figs. 7A, 7B, and 8. A processing flow of the subroutine for third index value calculation will be described later. After that, the control unit 201 ends the processing.

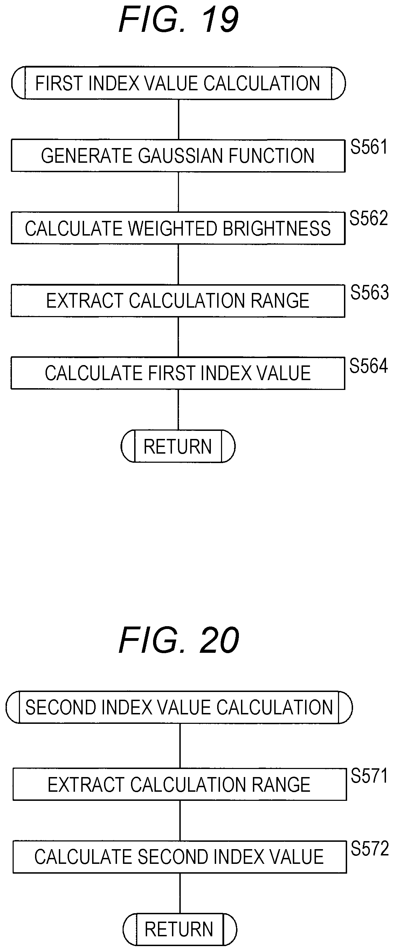

Fig. 19 is a flowchart for describing the processing flow of the subroutine for first index value calculation. The subroutine for first index value calculation is a subroutine for calculating a first index value described with reference to Figs. 4A, 4B, and 5A.

The control unit 201 generates a Gaussian function described with reference to Figs. 4B and Formula (2) (Step S561). The control unit 201 calculates weighted brightness Bw weighted by the Gaussian function described with reference to Formula (3) (Step S562). From the weighted brightness Bw, the control unit 201 extracts data included in the calculation range 47 described with reference to Fig. 5A (Step S563).

The control unit 201 calculates the first index value based on the data included in the calculation range 47 (Step S564). As described above, the first index value is, for example, a mean of the weighted brightness Bw. After that, the control unit 201 ends the processing.

Fig. 20 is a flowchart for describing the processing flow of the subroutine for second index value calculation. The subroutine for second index value calculation is a subroutine for calculating a second index value described with reference to Figs. 6A and 6B.

From the brightness data, the control unit 201 extracts data included in the calculation range 47 described with reference to Fig. 6A (Step S571). The control unit 201 calculates the second index value based on, for example, Formula (8) (Step S572). After that, the control unit 201 ends the processing.

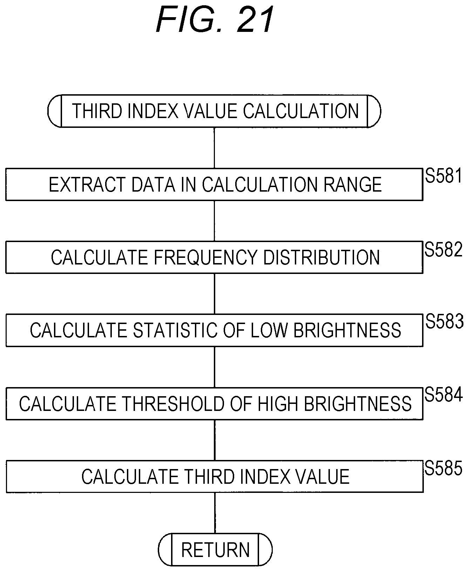

Fig. 21 is a flowchart for describing the processing flow of the subroutine for third index value calculation. The subroutine for third index value calculation is a subroutine for calculating a third index value described with reference to Figs. 7A, 7B, and 8.

From the brightness data, the control unit 201 extracts data included in the calculation range 47 described with reference to Fig. 7A (Step S581). The control unit 201 calculates a frequency distribution of the extracted data (Step S582). The calculation of the frequency distribution based on a large quantity of data is known, and the details thereof will be omitted herein.

The control unit 201 extracts data of the low brightness region 531 described with reference to Fig. 7B. The control unit 201 calculates two statistics, a mean $\mu L$ and a standard deviation $\sigma L$, of the extracted data (Step S583). Based on Formula (9), the control unit 201 calculates a second threshold, a threshold on the high brightness side (Step S584).

From the brightness data, the control unit 201 extracts data of the high brightness region 532 described with reference to Fig. 8. The control unit 201 calculates a third index value, a standard deviation $\sigma H$ of the extracted data (Step S585). After that, the control unit 201 ends the processing.

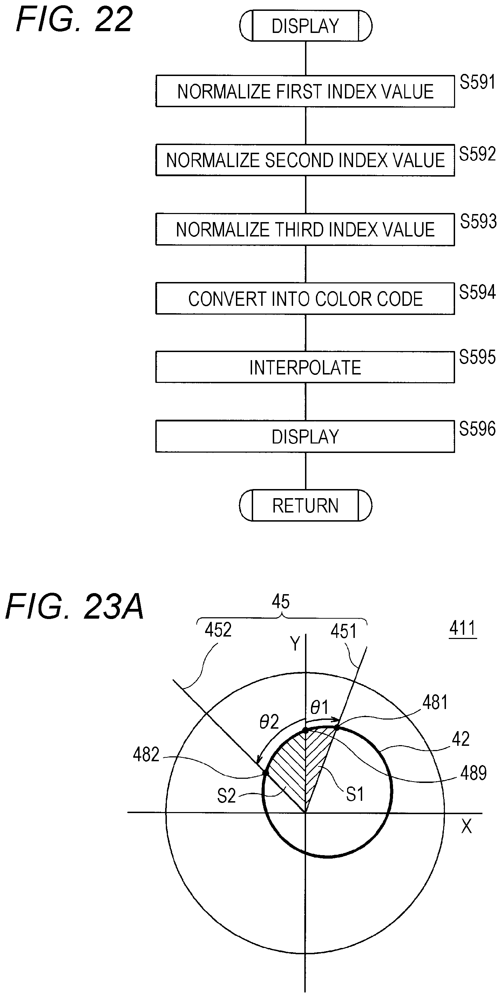

Fig. 22 is a flowchart for describing the processing flow of the subroutine for display. The subroutine for display is a subroutine for displaying the index value image 44 based on the index value DB 39.

From the first index value field of the index value DB 39, the control unit 201 acquires the first index value related to each scanning line 45 forming the batch of tomographic images 41. The control unit 201 normalizes each acquired first index value to an integer from 0 to 255 (Step S591).

From the second index value field of the index value DB 39, the control unit 201 acquires the second index value related to each scanning line 45 forming the batch of tomographic images 41. The control unit 201 normalizes each acquired second index value to an integer from 0 to 255 (Step S592).

From the third index value field of the index value DB 39, the control unit 201 acquires the third index value related to each scanning line 45 forming the batch of tomographic images 41. The control unit 201 normalizes each acquired third index value to an integer from 0 to 255 (Step S593).

For each scanning line 45, the control unit 201 converts the normalized first to third index values into a color code (Step S594). Specifically, the color code is expressed as RGB (AR, AG, AB) where an index value to be allocated to R (red), an index value to be allocated to G, and an index value to be allocated to B (blue) in the first, second, and third index values are denoted by AR, AG, and AB, respectively.

For each scanning line 45, the control unit 201 sets a color expressed by the color code at a positional coordinate determined by the length L recorded in the L field of the index value DB 39 and a tomographic position Z obtained by integrating the interval T with the tomogram number recorded in the tomogram number field. By interpolation, the control unit 201 determines the color code of a positional coordinate corresponding to a gap between the scanning lines 45 (Step S595).

The control unit 201 displays, on the display unit 205, an image generated by the color code of the positional coordinate (Step S596). According to instructions from a user, the control unit 201 rotates the image or scales the image up and down. The control unit 201 may display, on the display unit 205, an indicator, a handle, and the like that can be used when the user manipulates the image. After that, the control unit 201 ends the processing.

According to this embodiment, it is possible to generate an image including information on the size of the scanning lines 45. Among the scanning lines 45, the index value image 44 shows an index in a developed view focusing on information of a part near the representative line 42, which enables a user such as a doctor to intuitively understand the part that the user wants to pay attention to.

For example, in a case where the representative line 42 is set at a position corresponding to the lumen of a blood vessel, the length of the representative line 42 is represented by a vertical length of the index value image 44 illustrated in Fig. 1, whereby the user easily understands whether a region of interest is narrowed or expanded.

Three indices obtained by different calculation methods are allocated to R, G, and B, and the three indices are displayed on one index value image 44. Accordingly, the user can understand a lot of information in a short time. Note that the type of index to be used and the combination of indices and colors may be appropriately changed by the user.

The control unit 201 may display the index value image 44 and a tomographic image 41 side by side on the display unit 205. Alternatively, when the user clicks on one spot in the index value image 44, the control unit 201 may display a tomographic image 41 including the clicked spot on the display unit 205.

[Modification-2]

Basing the center point of the representative line 42, the control unit 201 may determine lengths L and ΔL along a tomographic image 41 described with reference to Fig. 10. Specifically, as described with reference to Fig. 10, the control unit 201 calculates an index value for an intersection, such as the first intersection 481 and the second intersection 482, between each scanning line 45 and the representative line 42.

The control unit 201 calculates the center point of the representative line 42. The control unit 201 determines ΔL at every constant angle from the center point and generates the index value stripe 43. That is to say, ΔL is a constant value over the entire representative line 42.

According to this modification, it is possible to provide the catheter system 10 that displays in an easy-to-understand manner the size of a blood vessel that changes due to stenosis or the like.

An angle between the first scanning line 451 and the third scanning line 453 is indicated by Δθ. In all the scanning lines 45 forming the X-Y format tomographic image 411, Δθ is constant. For example, when one tomographic image 41 includes five hundred and twelve scanning lines 45, Δθ is about 0.7 degrees. In Fig. 10, Δθ is schematically illustrated at a large angle. A length from the first intersection 481 to the third intersection 483 along the representative line 42 is indicated by ΔL.

The angle Δθ is small, and ΔL can be approximated by Formula (10).

$$\Delta L = R11 \times \sin\Delta\theta \cdots\cdots (10)$$

Similarly, an arc length ΔLn between the n-th scanning line 45 and the (n + 1)th scanning line 45 can be approximated by Formula (11).

$$\Delta Ln = R1n \times \sin\Delta\theta \cdots\cdots (11)$$

R1n is a distance between the center of the image-acquiring catheter 28 and the intersection 48 between the n-th scanning line 45 and the representative line 42.

The length L1 along the representative line 42 can be calculated by adding ΔL represented by Formula (10) while sequentially moving the scanning line 45 from the reference line to the first scanning line 451. Through these steps, the control unit 201 can generate the position-index value graph 55 based on the angle-index value graph 54. The control unit 201 converts index values on the ordinate of the position-index value graph 55 into colors as described above, thereby generating one index value stripe 43 corresponding to one tomographic image 41.

[Modification-3]

In this modification, an area is used instead of the length L along the arc of the representative line 42. Figs. 23A to 23C are views for describing a relation between an angle and an area according to this modification.

Fig. 23A schematically illustrates an image in which the representative line 42 is superimposed on the X-Y format tomographic image 411. In this modification, instead of the length L1 from the reference intersection 489 to the first intersection 481, a substantially fan-shaped area S1 surrounded by the representative line 42, the first scanning line 451, and the Y axis is used. Similarly, instead of the length L2 from the reference intersection 489 to the second intersection 482, a substantially fan-shaped area S2 surrounded by the representative line 42, the second scanning line 452, and the Y axis is used.

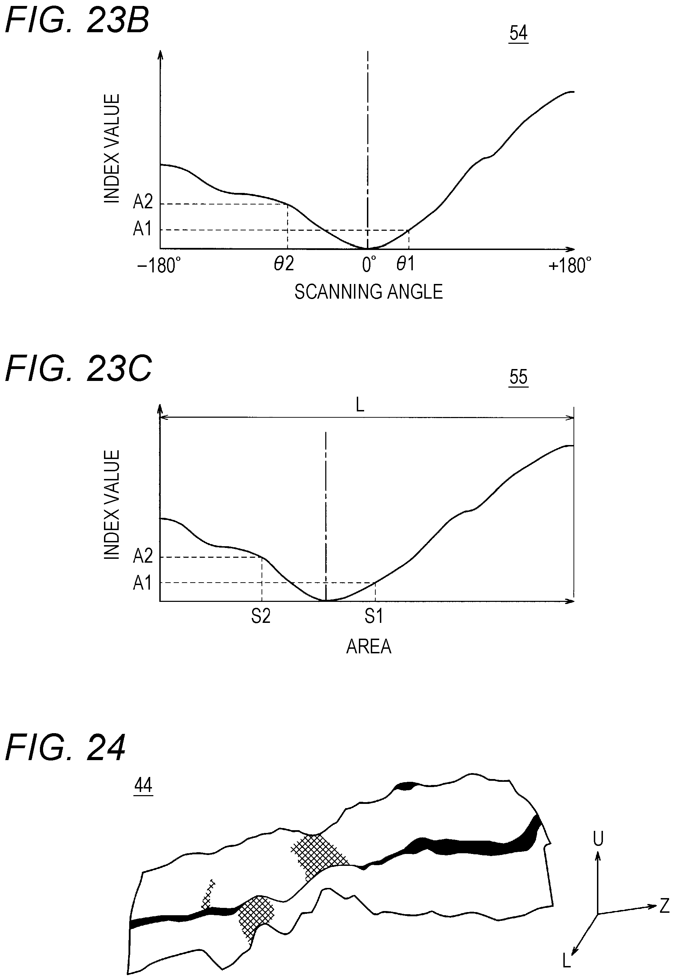

Fig. 23B illustrates the angle-index value graph 54 as in Fig. 9B. Fig. 23C illustrates an area-index value graph 56. In Fig. 23, the area described with reference to Fig. 23A is taken along the abscissa. In Fig. 23, the index value is taken along the ordinate.

As is clear from Fig. 23A, the scanning angle and the area are in a one-to-one correspondence. The area S1 can be calculated by, for example, a product of the number of pixels included in a part hatched downward to the left in Fig. 23A and an area of one pixel. Therefore, the control unit 201 can convert the abscissa of the angle-index value graph 54 into an area and create the area-index value graph 56.

Generating each index value stripe 43 based on the area-index value graph 56 makes it possible to generate the index value image 44 where an index value corresponding to each scanning line 45 is set at a positional coordinate determined by an area S and a tomographic position Z.

[Modification-4]

In this modification, index value stripes 43 with the same length are generated for each tomographic image 41, and then, the stripes are entirely elongated or contracted based on values related to the representative line 42.

Specifically, the control unit 201 does not create the position-index value graph 55 in the generation method described with reference to Fig. 3 but generates the index value stripes 43 based on the angle-index value graph 54. Each length of the index value stripes 43 generated herein corresponds to a 360-degree angle, and the index value stripes 43 are generated to have the same length for all the tomographic images 41.

The control unit 201 calculates values related to the representative line 42 for each tomographic image 41. The values related to the representative line 42 are, for example, a length of the representative line 42, an area inside the representative line 42, and a mean diameter of the representative line 42. The control unit 201 elongates and contracts the index value stripes 43 based on the values related to the representative line 42. Through these steps, the control unit 201 generates the index value stripes 43 having different lengths from one tomographic image 41 to another as described with reference to Fig. 1.

[Modification-5]

The control unit 201 may display an index on the index value image 44 based on the values related to the representative line 42. For example, on the edge of the index value image 44, the control unit 201 displays an index corresponding to a position of a tomographic image 41 having a representative line 42 with the smallest mean diameter. The control unit 201 may color an index value stripe 43 which corresponds to a tomographic image 41 employing a mean diameter of the representative line 42 and display the index value stripe 43, differently from the other index value stripes 43.

[Modification-6]

The control unit 201 may create a plurality of representative lines 42 and calculate index values. For example, the control unit 201 creates two representative lines 42, a representative line 42 corresponding to the lumen of a blood vessel and a representative line 42 corresponding to the external elastic lamina. The control unit 201 subtracts an area of the representative line 42 corresponding to the lumen of the blood vessel from an area of the representative line 42 corresponding to the external elastic lamina. In this manner, the control unit 201 can calculate an index value corresponding to a plaque area for each tomographic image 41.

[Second Embodiment]

Fig. 24 is an example of a screen according to a second embodiment. A control unit 201 sets a color corresponding to an index value at a positional coordinate determined by a length L and a three-dimensional space obtained by adding a third axis U to a tomographic position Z. An index value image 44 is displayed in a curved form in the three-dimensional space.

The third axis U is, for example, a distance from the center of the X-Y format tomographic image 411 in Fig. 9A, that is, the center of the image-acquiring catheter 28, to the intersection 48. Other optional parameters may be set as the third axis U.

According to this modification, it is possible to provide an information processing apparatus 200 that displays the index value image 44 representing more information simultaneously.

[Third Embodiment]

This embodiment relates to a catheter system 10 that acquires a tomographic image 41 in real time and displays an index value image 44. The same parts as those in the first embodiment will be omitted.

Fig. 25 is a view for describing a configuration of the catheter system 10 according to the third embodiment. The catheter system 10 includes an image processing apparatus 210, a catheter control device 27, a motor driving unit (MDU) 289, and an image-acquiring catheter 28. The image-acquiring catheter 28 is connected to the image processing apparatus 210 through the MDU 289 and the catheter control device 27.

The image processing apparatus 210 includes a control unit 211, a primary storage device 212, a secondary storage device 213, a communication unit 214, a display unit 215, an input unit 216, and a bus. The control unit 211 is an arithmetic control device that executes a program according to this embodiment. The control unit 211 employs, for example, one or more CPUs or GPUs, or a multi-core CPU. The control unit 211 is connected to each hardware component constituting the image processing apparatus 210 through the bus.

The primary storage device 212 is a storage device such as an SRAM, a DRAM, and a flash memory. The primary storage device 212 temporarily stores information necessary during processing by the control unit 211 and a program being executed by the control unit 211.

The secondary storage device 213 is a storage device such as an SRAM, a flash memory, a hard disk, and a magnetic tape. The secondary storage device 213 stores a tomographic image DB 36, a maximum gradient point DB 37, a representative line DB 38, an index value DB 39, programs to be executed by the control unit 211, and various kinds of data for executing the programs. The tomographic image DB 36, the maximum gradient point DB 37, the representative line DB 38, and the index value DB 39 may be stored in an external mass-storage device or the like connected to the image processing apparatus 210. The external mass-storage device may be what is called a cloud storage.

The communication unit 214 is an interface that conducts communication between the image processing apparatus 210 and a network. The display unit 215 is, for example, a liquid crystal display panel or an organic EL panel. The input unit 216 is, for example, a keyboard or a mouse. The input unit 216 may be layered on the display unit 215 to constitute a touch panel. The display unit 215 may be a display apparatus connected to the image processing apparatus 210.

The image processing apparatus 210 is a general-purpose personal computer, a tablet, a large computing machine, or a virtual machine that runs on a large computing machine. The image processing apparatus 210 may include a plurality of decentralized personal computers or hardware such as a large computing machine. The image processing apparatus 210 may include a cloud computing system. The image processing apparatus 210 and the catheter control device 27 may constitute integrated hardware.

The image-acquiring catheter 28 includes a sheath 281, a shaft 283 inserted into the sheath 281, and a sensor 282 disposed at a distal end of the shaft 283. The MDU 289 causes the shaft 283 and the sensor 282 to rotate and move forward and backward inside the sheath 281.

The sensor 282 is, for example, an ultrasonic transducer that transmits and receives ultrasonic waves or a transmitter/receiver for optical coherence tomography (OCT) that emits near-infrared light and receives reflected light. Hereinafter illustrated is a case where the image-acquiring catheter 28 is an intravascular ultrasound (IVUS) catheter used for capturing an ultrasound tomographic image from inside the circulatory organ.

The catheter control device 27 creates one tomographic image 41 per revolution of the sensor 282. The MDU 289 rotates the sensor 282 while pulling or pushing the sensor 282 in the axial direction, whereby the catheter control device 27 continuously creates a plurality of tomographic images 41 substantially perpendicular to the sheath 281.

The control unit 211 successively acquires the tomographic images 41 from the catheter control device 27 and records in the tomographic image DB 36. In this manner, what is called three-dimensional scanning is performed, and a batch of tomographic images 41 is recorded in the tomographic image DB 36.

The operation to move the sensor 282 forward and backward includes both the operation to move the entire image-acquiring catheter 28 forward and backward and the operation to move the sensor 282 forward and backward inside the sheath 281. The forward and backward movement may be automatically operated at a predetermined speed by the MDU 289 or may be manually operated by a user.

Note that the image-acquiring catheter 28 is not limited to a mechanical scanning catheter that mechanically rotates and moves forward and backward. For example, the image-acquiring catheter 28 may be an electronic radial scanning image-acquiring catheter using the sensor 282 having a plurality of annularly disposed ultrasound transducers.

The control unit 211 performs the processing described in the first embodiment using the tomographic image DB 36 recorded by the above operation and displays the index value image 44 on the display unit 215.

[Fourth Embodiment]

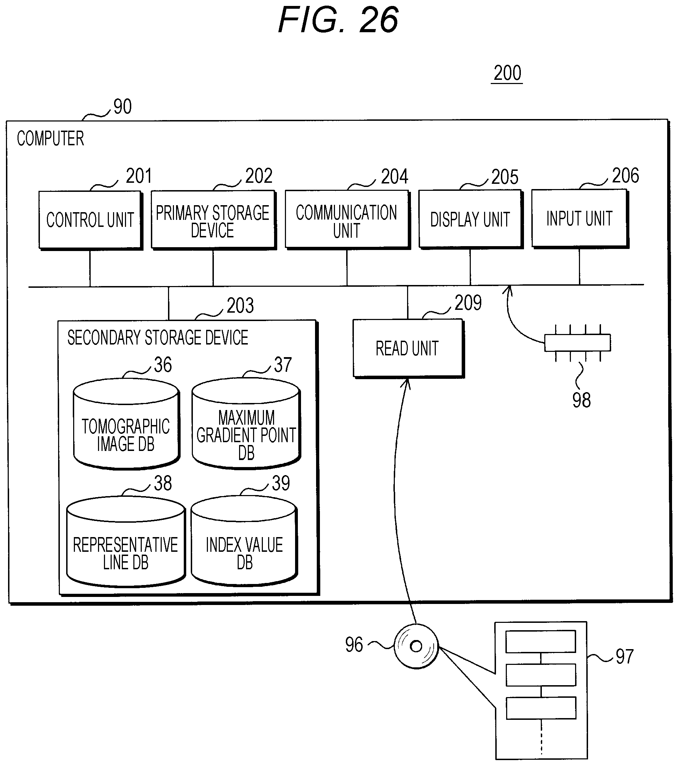

Fig. 26 is a view for describing a configuration of an information processing apparatus 200 according to a fourth embodiment. This embodiment relates to a mode that implements the information processing apparatus 200 of this embodiment by causing a general-purpose computer 90 and a program 97 to work in combination. The same parts as those in the first embodiment will be omitted.

In addition to the aforementioned control unit 201, primary storage device 202, secondary storage device 203, communication unit 204, display unit 205, input unit 206, and bus, the computer 90 includes a read unit 209.

The program 97 is recorded in a portable recording medium 96. The control unit 201 reads the program 97 through the read unit 209 and stores the program 97 in the secondary storage device 203. The control unit 201 may read out the program 97 stored in a semiconductor memory 98 such as a flash memory mounted in the computer 90. Alternatively, the control unit 201 may download the program 97 from another server computer (not illustrated) connected through the communication unit 204 and a network (not illustrated) and store the program 97 in the secondary storage device 203.

The program 97 is installed as a control program for the computer 90 and is loaded into the primary storage device 202 to be executed. In this manner, the information processing apparatus 200 described in the first embodiment is implemented. The program 97 in this embodiment is an example of a program product.

The technical features (components) described in the embodiments and modifications can be combined, which may create new technical features.

It should be understood that the embodiments disclosed herein are examples in all respects and are not restrictive. The scope of the present invention is indicated not by the above signification but by the claims and is intended to include all changes within the signification and scope equivalent to the claims.

Reference Signs List

| | |
|---|---|
| 10 | CATHETER SYSTEM |
| 200 | INFORMATION PROCESSING APPARATUS |
| 201 | CONTROL UNIT |
| 202 | PRIMARY STORAGE DEVICE |
| 203 | SECONDARY STORAGE DEVICE |
| 204 | COMMUNICATION UNIT |
| 205 | DISPLAY UNIT |
| 206 | INPUT UNIT |
| 209 | READ UNIT |
| 210 | IMAGE PROCESSING APPARATUS |
| 211 | CONTROL UNIT |
| 212 | PRIMARY STORAGE DEVICE |
| 213 | SECONDARY STORAGE DEVICE |
| 214 | COMMUNICATION UNIT |
| 215 | DISPLAY UNIT |
| 216 | INPUT UNIT |
| 27 | CATHETER CONTROL DEVICE |
| 28 | IMAGE-ACQUIRING CATHETER |
| 281 | SHEATH |
| 282 | SENSOR |
| 283 | SHAFT |
| 289 | MDU |
| 36 | TOMOGRAPHIC IMAGE DB |
| 37 | MAXIMUM GRADIENT POINT DB |
| 38 | REPRESENTATIVE LINE DB |
| 39 | INDEX VALUE DB |
| 41 | TOMOGRAPHIC IMAGE |
| 411 | X-Y FORMAT TOMOGRAPHIC IMAGE |
| 412 | R-T FORMAT TOMOGRAPHIC IMAGE |
| 42 | REPRESENTATIVE LINE |
| 422 | TEMPORARY REPRESENTATIVE LINE |
| 43 | INDEX VALUE STRIPE |
| 44 | INDEX VALUE IMAGE |
| 45 | SCANNING LINE |
| 451 | FIRST SCANNING LINE |

452     SECOND SCANNING LINE
453     THIRD SCANNING LINE
46      BLOCK
461     FIRST BLOCK
468     EIGHTH BLOCK
47      CALCULATION RANGE
48      INTERSECTION
481     FIRST INTERSECTION
482     SECOND INTERSECTION
489     REFERENCE INTERSECTION
51      GAUSSIAN FUNCTION GRAPH
52      RAYLEIGH DISTRIBUTION GRAPH
53      BRIGHTNESS HISTOGRAM
531     LOW BRIGHTNESS REGION
532     HIGH BRIGHTNESS REGION
54      ANGLE-INDEX VALUE GRAPH
55      POSITION-INDEX VALUE GRAPH
56      AREA-INDEX VALUE GRAPH
59      BRIGHTNESS GRAPH
90      COMPUTER
96      PORTABLE RECORDING MEDIUM
97      PROGRAM
98      SEMICONDUCTOR MEMORY

**Claims**

1. A program for causing a computer to execute processing comprising:

   acquiring a plurality of tomographic images generated by three-dimensional scanning using an image-acquiring catheter that acquires an image while moving a scanning plane in an axial direction;
   determining a representative line for each of the tomographic images;
   calculating an index value of a scanning line in a calculation range determined based on an intersection between the scanning line forming each of the tomographic images and the representative line; and
   displaying a color determined based on the index value at a coordinate determined by a position of each of the tomographic images and a position of the intersection.

2. The program according to claim 1,
   wherein the representative line is a closed curve surrounding the image-acquiring catheter.

3. The program according to claim 1 or 2,
   wherein the representative line has a circular shape surrounding the image-acquiring catheter.

4. The program according to any one of claims 1 to 3,
   wherein the coordinate is determined based on a position of each of the tomographic images and a value related to the representative line.

5. The program according to claim 4,
   wherein the value related to the representative line is a length along the representative line from a reference point determined on the representative line to the intersection.

6. The program according to any one of claims 1 to 5,
   wherein the index value is determined based on one or more of a first index value, a second index value, and a third index value calculated by different calculation methods.

7. The program according to claim 6,

   wherein the first index value is calculated by weighting a brightness value of the scanning line by a Gaussian function having the intersection as a peak position and

by averaging the weighted brightness value in the calculation range.

8. The program according to claim 6 or 7,
wherein the second index value is a function of a maximum likelihood estimate related to a parameter of a Rayleigh distribution that is estimated based on a brightness value of the scanning line in the calculation range.

9. The program according to any one of claims 6 to 8,

wherein the third index value is a second statistic obtained by
calculating a frequency distribution of a brightness value of the scanning line,
calculating a first statistic of the brightness value in a range of the frequency distribution where the brightness value is lower than a predetermined first threshold,
calculating a second threshold based on the first statistic calculated, and
calculating a second statistic in a range of the frequency distribution where the brightness value is higher than the second threshold.

10. The program according to claim 9,

wherein the first statistic is a mean and a standard deviation, and
the second statistic is a standard deviation.

11. The program according to any one of claims 6 to 10,

wherein the color determined based on the index value is generated by
normalizing each of the first index value, the second index value, and the third index value to an integer from 0 to 255, and
allocating the normalized first index value, second index value, and third index value, in any order without overlaps, to brightness of a plurality of color channels forming a color image.

12. The program according to any one of claims 1 to 11,
wherein the calculation range is a range including the intersection.

13. The program according to any one of claims 1 to 12,
wherein the calculation range is a range where a distance from the center of the image-acquiring catheter ranges inclusively from (R1 - b/2) to a maximum range where an image is acquired, R1 denoting the distance between the center of the image-acquiring catheter and the intersection and b denoting a predetermined constant.

14. The program according to any one of claims 1 to 12,
wherein the calculation range is a range where a distance from the center of the image-acquiring catheter ranges inclusively from (R1 - b/2) to (R1 + b), R1 denoting the distance between the center of the image-acquiring catheter and the intersection and b denoting a predetermined constant.

15. The program according to any one of claims 1 to 14,
wherein the program causes a computer to display a color determined based on the index value at a coordinate in a three-dimensional space determined by a third axis in addition to a position of each of the tomographic images and a position of the intersection.

16. The program according to claim 15,
wherein the third axis is a distance between the image-acquiring catheter and the intersection.

17. An information processing method executed by a computer, the method comprising:

acquiring a plurality of tomographic images generated by three-dimensional scanning using an image-acquiring catheter that acquires an image while moving a scanning plane in an axial direction;
determining a representative line for each of the tomographic images;
calculating an index value of a scanning line in a calculation range determined based on an intersection between the scanning line forming each of the tomographic images and the representative line; and
displaying a color determined based on the index value at a coordinate determined by a position of each of the tomographic images and a position of the intersection.

18. An information processing apparatus comprising a control unit,

wherein the control unit acquires a plurality of tomographic images generated by three-dimensional scanning using an image-acquiring catheter that acquires an image while moving a scanning plane in an axial direction; determines a representative line for each of the tomographic images; calculates an index value of a scanning line in a calculation range determined based on an intersection between the scanning line forming each of the tomographic images and the representative line; and displays a color determined based on the index value at a coordinate determined by a position of each of the tomographic images and a position of the intersection.

# FIG. 1

STICK TOGETHER

# FIG. 2

200

INFORMATION PROCESSING APPARATUS

201
**CONTROL UNIT**

202
**PRIMARY STORAGE DEVICE**

204
**COMMUNICATION UNIT**

205
**DISPLAY UNIT**

206
**INPUT UNIT**

203
SECONDARY STORAGE DEVICE

36
TOMOGRAPHIC IMAGE DB

37
MAXIMUM GRADIENT POINT DB

38
REPRESENTATIVE LINE DB

39
INDEX VALUE DB

# FIG. 3

41

411          412

ACQUIRE
TOMOGRAPHIC IMAGE

X-Y FORMAT

+180°

0°

−180°

R-T FORMAT

411

DETERMINE
REPRESENTATIVE LINE

42

45

48

X-Y FORMAT

+180°

0°

−180°

R-T FORMAT

412

48

45

42

CALCULATE
INDEX VALUE

54

INDEX VALUE

−180°       0°       +180°
SCANNING ANGLE

CONVERT

55

INDEX VALUE

ARC LENGTH

ALLOCATE TO COLOR

T

L

43

## FIG. 4A

## FIG. 4B

## FIG. 5A

## FIG. 5B

FIG. 6A

59

FIG. 6B

52

*FIG. 7A*

*FIG. 7B*

# FIG. 8

53

# FIG. 9A

## FIG. 9B

54

## FIG. 9C

55

# FIG. 10

# FIG. 11

412

−180°

0°

+180°

R-T FORMAT

SLICE →

412

461
462
463
464
465
466
467
468

} 46

CALCULATE MAXIMUM
GRADIENT POINT G

412

■ G1 — 461
■ G2 — 462
■ G3 — 463
■ G4 — 464
■ G5 — 465
■ G6 — 466
■ G7 — 467
■ G8 — 468

} 46

← CONVERT INTO
COORDINATES

411

G5
G4   G6
422 — G3  ×C  G7
G2      G8
G1

X-Y FORMAT

↓

CALCULATE CENTROID C

↓

CALCULATE DISTANCE D

↓

CALCULATE TEMPORARY
REPRESENTATIVE LINE

# FIG. 12

TEMPORARY REPRESENTATIVE LINE
OF n-th TOMOGRAPHIC IMAGE
· CENTROID Cn
· DISTANCE CENTRAL TENDENCY Dn

n          41

422

MEAN SHIFT OF PRECEDING AND
FOLLOWING K-FRAMES
· CENTROID Cnavg
· DISTANCE CENTRAL TENDENCY Dnavg

Dnavg

42

Cnavg

411

## FIG. 13A

| 3D SCANNING ID | V001 |
|---|---|

_36_

| | TOMOGRAPHIC IMAGE | |
|---|---|---|
| TOMOGRAM NUMBER | X-Y FORMAT | R-T FORMAT |
| 1 | XY001 | RT001 |
| 2 | XY002 | RT002 |
| 3 | XY003 | RT003 |
| 4 | XY004 | RT004 |
| 5 | XY005 | RT005 |

## FIG. 13B

_37_

| 3D SCANNING ID | V001 |
|---|---|
| TOMOGRAM NUMBER | 12 |
| DISTANCE CENTRAL TENDENCY | 220 |

| BLOCK NUMBER | MAXIMUM GRADIENT POSITION | | DISTANCE | FLAG |
|---|---|---|---|---|
| | R | T | | |
| 1 | 302 | 7.5 | 201 | 1 |
| 2 | 308 | 22.1 | 210 | 1 |
| 3 | 420 | 40.5 | 204 | 0 |
| 4 | 331 | 52.1 | 190 | 1 |
| ⋮ | ⋮ | ⋮ | ⋮ | ⋮ |

## FIG. 13C

_38_

| 3D SCANNING ID | V001 |
|---|---|

| TOMOGRAM NUMBER | TEMPORARY REPRESENTATIVE LINE | | | REPRESENTATIVE LINE | | |
|---|---|---|---|---|---|---|
| | CENTROID | | DISTANCE CENTRAL TENDENCY | CENTROID | | DISTANCE CENTRAL TENDENCY |
| | X | Y | | X | Y | |
| 1 | 10 | 10 | 158 | 10 | 10 | 158 |
| 2 | 12 | 11 | 165 | 12 | 11 | 165 |
| ⋮ | ⋮ | ⋮ | ⋮ | ⋮ | ⋮ | ⋮ |

# FIG. 14

| 3D SCANNING ID | V001 |
|---|---|
| TOMOGRAM NUMBER | 12 |

39

| SCANNING LINE NUMBER | INTERSECTION | | INDEX VALUE | | |
|---|---|---|---|---|---|
| | R1 | L | FIRST INDEX VALUE | SECOND INDEX VALUE | THIRD INDEX VALUE |
| 1 | 200 | 0.00 | 100 | 250 | 10 |
| 2 | 215 | 2.64 | 120 | 240 | 25 |
| 3 | 210 | 5.22 | 85 | 222 | 15 |
| ⋮ | ⋮ | ⋮ | ⋮ | ⋮ | ⋮ |

## FIG. 15

```
                    ┌─────────┐
                    │  START  │
                    └────┬────┘
                         │◄──────────────────────┐
        ┌────────────────┴────────────────┐      │
        │  ACQUIRE TOMOGRAPHIC IMAGE       │ S501 │
        └────────────────┬────────────────┘      │
        ┌────────────────┴────────────────┐      │
        │  CALCULATE TEMPORARY            │ S502  │
        │  REPRESENTATIVE LINE             │      │
        └────────────────┬────────────────┘      │
                   S504  │                        │
                    ◄────┴────►   NO              │
                   COMPLETE? ──────────────────────┘
                    ◄────┬────►
                     YES │
        ┌────────────────┴────────────────┐
        │ CALCULATE REPRESENTATIVE LINE    │ S505
        └────────────────┬────────────────┘
                         │◄──────────────────────┐
        ┌────────────────┴────────────────┐      │
        │  SELECT TOMOGRAPHIC IMAGE        │ S506 │
        └────────────────┬────────────────┘      │
                         │◄───────────────────┐   │
        ┌────────────────┴────────────────┐   │   │
        │  SELECT SCANNING LINE            │ S507  │
        └────────────────┬────────────────┘   │   │
        ┌────────────────┴────────────────┐   │   │
        │  CALCULATE INDEX VALUE           │ S508  │
        └────────────────┬────────────────┘   │   │
        ┌────────────────┴────────────────┐   │   │
        │  RECORD                          │ S510  │
        └────────────────┬────────────────┘   │   │
                   S511  │                     │   │
                    ◄────┴────►   NO           │   │
                   COMPLETE? ───────────────────┘   │
                    ◄────┬────►                     │
                     YES │                          │
                   S512  │                          │
                    ◄────┴────►   NO                │
                   COMPLETE? ───────────────────────┘
                    ◄────┬────►
                     YES │
        ┌────────────────┴────────────────┐
        │  DISPLAY                         │ S513
        └────────────────┬────────────────┘
                    ┌─────┴─────┐
                    │    END    │
                    └───────────┘
```

# FIG. 16

```
        ┌─────────────────────────┐
        │       TEMPORARY         │
        │  REPRESENTATIVE LINE     │
        │     CALCULATION         │
        └─────────────────────────┘
                    │
        ┌─────────────────────────┐
        │   SLICE INTO BLOCKS     │  S521
        └─────────────────────────┘
                    │
                    ▼◄─────────────────────────┐
        ┌─────────────────────────┐            │
        │     SELECT BLOCK        │  S522      │
        └─────────────────────────┘            │
                    │                          │
        ┌─────────────────────────┐            │
        │  CALCULATE MAXIMUM      │  S523      │
        │  GRADIENT POINT         │            │
        └─────────────────────────┘            │
                    │         S524             │
                    ◇─────────────── NO ───────┘
              COMPLETE?
                    │ YES
        ┌─────────────────────────┐
        │  CALCULATE CENTROID     │  S525
        └─────────────────────────┘
                    │
        ┌─────────────────────────┐
        │  CALCULATE DISTANCE     │  S526
        └─────────────────────────┘
                    │         S527
                    ◇─────────────── NO ───────┐
              FIRST FRAME?                      │
                    │ YES                       │
                    │                  ┌──────────────────┐
                    │                  │ EXCLUDE OUTLIER  │ S531
                    │                  └──────────────────┘
                    │                       │
        ┌──────────────────┐     ┌──────────────────┐
        │   CALCULATE      │S528 │   CALCULATE      │ S532
        │ CENTRAL TENDENCY │     │ CENTRAL TENDENCY │
        └──────────────────┘     └──────────────────┘
                    │◄───────────────────┘
        ┌─────────────────────────┐
        │        RECORD           │  S533
        └─────────────────────────┘
                    │
             ( RETURN )
```

38

# FIG. 17

```
    ┌─────────────────────────┐
    │  REPRESENTATIVE LINE     │
    │      CALCULATION         │
    └─────────────────────────┘
                │
    ┌─────────────────────────┐  S541
    │  ACQUIRE INFORMATION     │
    │    ON TEMPORARY          │
    │  REPRESENTATIVE LINE     │
    └─────────────────────────┘
                │
    ┌──────────────────────────┐ S542
    │  SELECT TOMOGRAM NUMBER   │
    └──────────────────────────┘
                │
             S543
          ╱  NEAR EITHER  ╲   NO
         ╲     END?        ╱ ─────────┐
           YES                        │
            │                   ┌──────────────────────┐ S545
            │                   │  CALCULATE MEAN SHIFT │
            │                   └──────────────────────┘
    ┌──────────────────────┐ S544    ┌──────────────────────┐ S546
    │  RECORD INFORMATION   │         │  RECORD INFORMATION   │
    │ ON REPRESENTATIVE LINE│         │ ON REPRESENTATIVE LINE│
    └──────────────────────┘         └──────────────────────┘
            │
        S547
      ╱ COMPLETE? ╲  NO
     ╲            ╱ ────
        YES
         │
    ┌──────────┐
    │  RETURN  │
    └──────────┘
```

39

# FIG. 18

INDEX VALUE CALCULATION

CALCULATE INTERSECTION · S551

CALCULATE ΔL · S552

CALCULATE L · S553

ACQUIRE SCANNING LINE DATA · S554

CALCULATE FIRST INDEX VALUE · S555

CALCULATE SECOND INDEX VALUE · S556

CALCULATE THIRD INDEX VALUE · S557

RETURN

# FIG. 19

```
( FIRST INDEX VALUE CALCULATION )
            │
┌──────────────────────────────────┐
│   GENERATE GAUSSIAN FUNCTION     │ S561
└──────────────────────────────────┘
            │
┌──────────────────────────────────┐
│  CALCULATE WEIGHTED BRIGHTNESS   │ S562
└──────────────────────────────────┘
            │
┌──────────────────────────────────┐
│    EXTRACT CALCULATION RANGE     │ S563
└──────────────────────────────────┘
            │
┌──────────────────────────────────┐
│   CALCULATE FIRST INDEX VALUE    │ S564
└──────────────────────────────────┘
            │
        ( RETURN )
```

# FIG. 20

```
( SECOND INDEX VALUE CALCULATION )
            │
┌──────────────────────────────────┐
│    EXTRACT CALCULATION RANGE     │ S571
└──────────────────────────────────┘
            │
┌──────────────────────────────────┐
│  CALCULATE SECOND INDEX VALUE    │ S572
└──────────────────────────────────┘
            │
        ( RETURN )
```

# FIG. 21

```
        ( | THIRD INDEX VALUE CALCULATION | )
                          |
    ┌─────────────────────────────────────────┐
    │   EXTRACT DATA IN CALCULATION RANGE      │ S581
    └─────────────────────────────────────────┘
                          |
    ┌─────────────────────────────────────────┐
    │   CALCULATE FREQUENCY DISTRIBUTION       │ S582
    └─────────────────────────────────────────┘
                          |
    ┌─────────────────────────────────────────┐
    │   CALCULATE STATISTIC OF LOW BRIGHTNESS  │ S583
    └─────────────────────────────────────────┘
                          |
    ┌─────────────────────────────────────────┐
    │ CALCULATE THRESHOLD OF HIGH BRIGHTNESS   │ S584
    └─────────────────────────────────────────┘
                          |
    ┌─────────────────────────────────────────┐
    │      CALCULATE THIRD INDEX VALUE         │ S585
    └─────────────────────────────────────────┘
                          |
                  ( | RETURN | )
```

## FIG. 22

DISPLAY

NORMALIZE FIRST INDEX VALUE — S591

NORMALIZE SECOND INDEX VALUE — S592

NORMALIZE THIRD INDEX VALUE — S593

CONVERT INTO COLOR CODE — S594

INTERPOLATE — S595

DISPLAY — S596

RETURN

## FIG. 23A

## FIG. 23B

<u>54</u>

## FIG. 23C

<u>55</u>

## FIG. 24

<u>44</u>

# FIG. 25

10

### 210

**IMAGE PROCESSING APPARATUS**

| 211 | 212 | 214 | 215 | 216 |
|---|---|---|---|---|
| CONTROL UNIT | PRIMARY STORAGE DEVICE | COMMUNICATION UNIT | DISPLAY UNIT | INPUT UNIT |

### 213

**SECONDARY STORAGE DEVICE**

| 36 | 37 | 38 | 39 |
|---|---|---|---|
| TOMOGRAPHIC IMAGE DB | MAXIMUM GRADIENT POINT DB | REPRESENTATIVE LINE DB | INDEX VALUE DB |

CATHETER CONTROL DEVICE 27

282    281    289

MDU

283

28

# FIG. 26

200

90

COMPUTER

| 201 | 202 | 204 | 205 | 206 |
|---|---|---|---|---|
| CONTROL UNIT | PRIMARY STORAGE DEVICE | COMMUNICATION UNIT | DISPLAY UNIT | INPUT UNIT |

203

SECONDARY STORAGE DEVICE

36

TOMOGRAPHIC IMAGE DB

37

MAXIMUM GRADIENT POINT DB

38

REPRESENTATIVE LINE DB

39

INDEX VALUE DB

209

READ UNIT

98

96

97

**INTERNATIONAL SEARCH REPORT**

| International application No. |
| --- |
| **PCT/JP2023/008441** |

**A.    CLASSIFICATION OF SUBJECT MATTER**

***A61B 8/12***(2006.01)i
FI:    A61B8/12

According to International Patent Classification (IPC) or to both national classification and IPC

**B.    FIELDS SEARCHED**

Minimum documentation searched (classification system followed by classification symbols)

A61B8/12

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2022-520409 A (KONINKLIJKE PHILIPS N.V.) 30 March 2022 (2022-03-30) <br> entire text, all drawings | 1-18 |
| A | JP 2021-41029 A (TERUMO CORP.) 18 March 2021 (2021-03-18) <br> entire text, all drawings | 1-18 |
| A | WO 2022/054805 A1 (TERUMO CORP.) 17 March 2022 (2022-03-17) <br> entire text, all drawings | 1-18 |
| A | JP 2021-531119 A (PULSE MEDICAL IMAGING TECHNOLOGY (SHANGHAI) CO., LTD.) 18 November 2021 (2021-11-18) <br> entire text, all drawings | 1-18 |

☐ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **17 April 2023** | **16 May 2023** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

<table>
<tr><td colspan="2" align="center">INTERNATIONAL SEARCH REPORT<br>Information on patent family members</td><td colspan="2">International application No.<br>**PCT/JP2023/008441**</td></tr>
</table>

| Patent document cited in search report | Publication date (day/month/year) | Patent family member(s) | Publication date (day/month/year) |
|---|---|---|---|
| JP 2022-520409 A | 30 March 2022 | US 2022/0142606 A1<br>entire text, all drawings<br>WO 2020/165389 A1<br>EP 3923818 A1<br>CN 113453628 A | |
| JP 2021-41029 A | 18 March 2021 | US 2022/0192631 A1<br>entire text, all drawings<br>WO 2021/048834 A1<br>EP 4014885 A1<br>CN 114650778 A | |
| WO 2022/054805 A1 | 17 March 2022 | (Family: none) | |
| JP 2021-531119 A | 18 November 2021 | US 2021/0298706 A1<br>entire text, all drawings<br>WO 2020/019408 A1<br>EP 3828815 A1<br>CN 109166101 A<br>CN 113012108 A<br>CN 113012109 A | |

Form PCT/ISA/210 (patent family annex) (January 2015)

**REFERENCES CITED IN THE DESCRIPTION**

**Patent documents cited in the description**

• JP 2021041029 A